# EUROPEAN PATENT APPLICATION

(11) **EP 3 950 954 A1**
(43) Date of publication of application: **09.02.2022**
(21) Application number: 19894068.6
(22) Date of filing: 05.12.2019
(51) Int. Cl.: C12Q 1/04, C12Q 1/68, C12Q 1/6886, G01N 33/50

(54) **METHOD FOR DETERMINING PROGNOSIS OF ENDOMETRIAL CANCER**

(30) Priority: 05.12.2018 JP 2018228276
(71) Applicant: Keio University, Tokyo, 108-8345 (JP); Sekisui Medical Co., Ltd., Tokyo 103-0027 (JP)
(72) Inventor: KANAI, Yae, Tokyo 160-8582 (JP); ARAI, Eri, Tokyo 160-8582 (JP); AOKI, Daisuke, Tokyo 160-8582 (JP); SUSUMU, Nobuyuki, Tokyo 160-8582 (JP); YAMAGAMI, Wataru, Tokyo 160-8582 (JP); MAKABE, Takeshi, Tokyo 160-8582 (JP)
(74) Representative: Schiener, Jens
(86) International application number: PCT/JP2019/047656
(87) International publication number: WO 2020/116573

(57) **Abstract**

Provided are a method for determining prognosis of endometrial cancer, a method for determining endometrial cancer patient compatible to preservation therapy, and a method for determining risk of endometrial cancer. The methods comprise detecting DNA methylation level at a target CpG site in a genomic DNA derived from an endometrial cell, endometrial cancer cell or tissue containing the cell. Prognosis of endometrial cancer, compatibility to preservation therapy, or risk of endometrial cancer of a subject is determined on the basis of the detected DNA methylation level.

SEQUENCE LISTING

<110>  KEIO\201@UNIVERSITY
       SEKISUI MEDICAL CO., LTD.

<120>  Method of determinating prognosis of endometrial cancer

<130>  DC0119

<150>  JP 2018-228276
<151>  2018-12-05

<160>  68

<170>  PatentIn version 3.5

<210>  1
<211>  122
<212>  DNA
<213>  Homo sapiens

<400>  1
gccgcgctcc cggcccggct cagccaggtg cccccacgcg gcagcgcccg gtctgcccgc      60

cggccccctg cgaacccctc gcctcacgcc cgcctgcctg cgcgcacgca cgcgcacgca      120

cg                                                                    122


<210>  2
<211>  122
<212>  DNA
<213>  Homo sapiens

<400>  2
gctcgcggcg tgcgcagggt cctcgtgctg ctattaacgt atttgcccat ttgtaaagca      60

cgattttaag ggatggtgag ttaaacaccg ctttttcggg gagggtgacg gggcgtggga      120

gg                                                                    122


<210>  3
<211>  122
<212>  DNA
<213>  Homo sapiens

<400>  3
agcagctagg cgctgggatc ggggtcggct ctcggctcgg ctcgcgggct gtaaggtctc      60

cgcggacccc accgcctctt ctgcgcgcta agggtgagcg ccaagcaatt tcgcgagcaa      120

ta                                                                    122


<210>  4
<211>  122
<212>  DNA
<213>  Homo sapiens

<400>  4
aatataagtc ctaaaaacag gcgcacccta atcctaccta cttcccacaa gaagccttca      60

cgcagccaag gcccgcccca catagcacac gcgcggattc gttttccgtt tggtaactga 120

ca 122

<210> 5
<211> 122
<212> DNA
<213> Homo sapiens

<400> 5
agtcgtccag gcggattcct ttttcgccag gtaaggctgg cccgggtgca tggggcccgg 60

cgtgccctgg gtaaggctgg cccaggcgcg tggggtccgg ggcgcgccgg gtaaggctgg 120

gc 122

<210> 6
<211> 122
<212> DNA
<213> Homo sapiens

<400> 6
tgactaaaga tgcaagtgcc taaaaggttg cgttgggcag gaggcgcttg gagggcagcg 60

cgggttccca gccattcctt gggtccaggt cgaggaaggc ctcgggagac gagtcccaca 120

gt 122

<210> 7
<211> 122
<212> DNA
<213> Homo sapiens

<400> 7
caaaagtaat ttttcagaat tttcagcttt ttcttattgt aggcatggga ataagaactt 60

cgaagctctt acatgttgga gccaaaattg gaattcccat gcatggggtt atgataaata 120

gt 122

<210> 8
<211> 122
<212> DNA
<213> Homo sapiens

<400> 8
ccagtgcaaa acaatgtctg ggcatcactg ctataagcat ttcataccta atccatcttt 60

cgagatagaa accttttaat gcttccatcc agttgatttt ctcattttct ccaaatatct 120

tc 122

<210> 9
<211> 122
<212> DNA
<213> Homo sapiens

<400> 9

attgttttaa aattcagatt tttaatagaa gtccttttct tactatatac ccagaatcaa 60

cgaagtcaaa aatcaaagtc tttgccacgg tttagaatca tatataaaat gatagctaca 120

tg 122


<210> 10
<211> 122
<212> DNA
<213> Homo sapiens

<400> 10
tctgacacac ctttctaaac ttcggcaaat caaatttacc ttttattaga catagagaat 60

cgctaattgt gtgaaacaaa tattgccctt cttctggata attcctataa atcatcttct 120

tt 122


<210> 11
<211> 122
<212> DNA
<213> Homo sapiens

<400> 11
tttgtggtac taggtaaaaa gcatacacat catggcaagg gagaattcga ccttcaactc 60

cgacttcatc ctcctgggaa tcttcaatca cagccccacc cacaccttcc tcttctttct 120

gg 122


<210> 12
<211> 122
<212> DNA
<213> Homo sapiens

<400> 12
agctggagga ggaactaact gtaagtgtgt ctcgaacttg cgcgggggct tcagcccgcc 60

cggggctgcg gagctggccg gtaaaggctc tcgggagagg ggcgcggtgt ctccacggtg 120

ac 122


<210> 13
<211> 122
<212> DNA
<213> Homo sapiens

<400> 13
cggacgccac ctaccacact atgaatacca tcccgtgcac gtcggccgcc tcttcttcat 60

cggtgcccat ctcgcaccct tccgcgttgg cgggcacgca ccaccaccac caccatcacc 120

ac 122


<210> 14
<211> 122
<212> DNA
<213> Homo sapiens

<400>  14
caaccggcag cgtccagctc ccgcacctcg ctgcacatcg cacctgagcc ccgccgcgac      60

cgcatcgcgc tcgctgcgac ccattcagac cccccagaaa cgccaagccg ctcccgctct      120

ag                                                                      122


<210>  15
<211>  122
<212>  DNA
<213>  Homo sapiens

<400>  15
gaaaaaaaaa aggcccttaa aactctgacc tggagcctcc cggctcttcc tcactgcacc      60

cgcaagcgcg ctaagggaga cgggaggcac aagcaagtcc caaacctttc tctgggtttt      120

ac                                                                      122


<210>  16
<211>  122
<212>  DNA
<213>  Homo sapiens

<400>  16
tgggattccc tgcgcagcca acaacagaaa gaagaaaacc agctcccaca cagagcctcc      60

cggctgcgca gacctttccc agcacagcgg attgccagct ccgagacccg ggactcctcc      120

tg                                                                      122


<210>  17
<211>  122
<212>  DNA
<213>  Homo sapiens

<400>  17
gggaaggggt agagggggcg gcgggggtcg ctccaaagac ttgtatttcg cgtttgcctc      60

cgggagctgg gagtaaggcc ttggatggcg ccgacgcggt tgcgaggaag ctgaggcctg      120

gg                                                                      122


<210>  18
<211>  122
<212>  DNA
<213>  Homo sapiens

<400>  18
attgtagtat tgttctcacc ctcacttttc ccattaattg gaatcaccaa tatcacatac      60

cgatgacaga tacgagcaga aatgcgttcg ccaaatatat cgactatatt gttcggagtg      120

tt                                                                      122


<210>  19
<211>  122

<212>  DNA
<213>  Homo sapiens

<400>  19
aatacgaggc cgagggagga attctgtcgt ctgggaacgg ctggtttcgc ggtcagacgc      60

cgagggccct cgaagtccct agcaacggct gcggagggag atgtaagtcg ttttggacgc     120

ag                                                                    122


<210>  20
<211>  122
<212>  DNA
<213>  Homo sapiens

<400>  20
agtacaagcg gaggactcgc caggcgctga gccggaatgt ggactgtacc acagtgactg      60

cgaagggagg ggaagacgac ggcaaggagg cctactacac gagcagtaag gtccacactt     120

tt                                                                    122


<210>  21
<211>  122
<212>  DNA
<213>  Homo sapiens

<400>  21
tgcacaactt ggataatcaa gtaaattata aagcaaattt cagtactctt gcctcagaac      60

cgctgccatc tctgcacctg taatgtagac agtgaaaacg ctggttattt attttgtact     120

tt                                                                    122


<210>  22
<211>  122
<212>  DNA
<213>  Homo sapiens

<400>  22
aaggagccgg aagagggcag agttaccaaa tgggctcctt agtcatggct tggggctcca      60

cgaccctcct ggaagcccgg agcctgggtg ggatagcgag gctgcgcgcg gccggcgccc     120

cg                                                                    122


<210>  23
<211>  122
<212>  DNA
<213>  Homo sapiens

<400>  23
aaagctattt aacaattaat gcgttcctgg gggaggccgg gggaagtacc cgccccttgt      60

cgggacgcaa agccaggcgt caggtccaaa ggggctgcag tgcagtccga tttcagcacg     120

ga                                                                    122

<210>  24
<211>  122
<212>  DNA
<213>  Homo sapiens

<400>  24
cggagcgcag cggaggggag cgctgggggg cggggagcgc tcgggcagcc accctgtccc      60

cgtctggagc cccgcgctgc gtctaggagg gcgcaacacg cagtccccgc gggggccaga      120

gc                                                                    122


<210>  25
<211>  122
<212>  DNA
<213>  Homo sapiens

<400>  25
agagggaggg tgtgaccatg gcccggagct ctccgaaagg ctgtgcggat tgctcggtgg      60

cgggatgtgg agcgcgtctt ctatgatgcc aggtgctggc caagcgctcg atgcaggctg      120

ct                                                                    122


<210>  26
<211>  122
<212>  DNA
<213>  Homo sapiens

<400>  26
cagccgccca ggcttcgggg aaggagggag gggagaataa tgcgcccctc tttcaataaa      60

cgccactgcc aaccactcca acagacacac tttcggtccc ccgccagagc tccggtgccc      120

cc                                                                    122


<210>  27
<211>  122
<212>  DNA
<213>  Homo sapiens

<400>  27
cacgcccgag ccacccgagg agtacgtgta cccggactac cgtggcaagg gctgcgtgga      60

cgagagcggc ttcgtgtacg cgatcgggga gaagttcgcg ccgggcccct cggcctgccc      120

gt                                                                    122


<210>  28
<211>  122
<212>  DNA
<213>  Homo sapiens

<400>  28
tgtcctgccc tgccctgccc tgcgcgcgca ccgcactcac acgtacaccc ggtcctcgca      60

cgcgcacaca cgcacactgt tccccgccgc tctaccctct agactgcaag ggaaacggct      120

cc                                                                    122

<210>  29
<211>  122
<212>  DNA
<213>  Homo sapiens

<400>  29
ccgccgggtg gggtcgggcc actgtaggct ggagcggggt tggatcaaat gcctttggcg      60

cgggaactga gtgctggccc gggagaccct ccggagagct cgcgggctcg gcctcggcct     120

cg                                                                    122


<210>  30
<211>  122
<212>  DNA
<213>  Homo sapiens

<400>  30
tgaggtcgat cttcagggcc agctcctccc gagtgtagat gtcggggtag tgagtctccg      60

cgaagaccct ttccagctct ttgagctggg cactggtgaa agtggtgcgg atgcgccgct     120

gc                                                                    122


<210>  31
<211>  122
<212>  DNA
<213>  Homo sapiens

<400>  31
tttcttattt ccgagaaatc tcgcaaacgg cgtgaacact acgctgattc cagaaatggt      60

cgggttccct tccttcactc aggatggtcg cctgctggga gctcatttcg aaccgagctg     120

aa                                                                    122


<210>  32
<211>  122
<212>  DNA
<213>  Homo sapiens

<400>  32
aacagaagtg gctctgaagt gcactggatc ttaggagaaa gcgagtcaaa aatgtttcaa      60

cggcctagac aatctgaaag ggggaagtcg attcataact gaagttattt cagcccctta     120

at                                                                    122


<210>  33
<211>  122
<212>  DNA
<213>  Homo sapiens

<400>  33
tgatcagaat atctgagaat caaatctttg tgatagatat atgaatggta cccgttacaa      60

cggatccttg tgggaaatat ggggtttaaa atattcaaat tgttattatc tagaaatgat     120

gt                                                                 122

<210>  34
<211>  122
<212>  DNA
<213>  Homo sapiens

<400>  34
aaccaaatca gcatgtaatg caaattacct cacatgaatt agtataagat ttaaataggt      60

cgtctttatg ttatgtgtta tggagattgt tgaaaattca ccctgaaaca cttggaagtg     120

ct                                                                 122

<210>  35
<211>  122
<212>  DNA
<213>  Homo sapiens

<400>  35
gtgtgagttt catgtcctca gtgcccatgt ctaaaagcaa aagtgaaaat taaatcagag      60

cggaaaacac ctctctattc aacaccagat tgttagggct gagctgaaaa gctcctaagt     120

aa                                                                 122

<210>  36
<211>  122
<212>  DNA
<213>  Homo sapiens

<400>  36
ttggggtgag accattcgtg gtgctggtgg gggccgtccc gtgtattgta ggatggttag      60

cggcatctgt ggtctccatc ctctaggtgc cattcccccc tcccagctat ggctacccca     120

ga                                                                 122

<210>  37
<211>  122
<212>  DNA
<213>  Homo sapiens

<400>  37
gtggggactg tggagaagtg gggaatgcag gtcccacaca cagcagcagc cctgactcag      60

cgccagccag taggtgccaa gtggtaacac aggcccccag tggccatatc tgatttttca     120

ag                                                                 122

<210>  38
<211>  122
<212>  DNA
<213>  Homo sapiens

<400>  38
tccataaaat tcagagtata ggactggtga gttttctctt cgttcttgtt aaccatatat      60

cgagttgtta tttgtagcta tagtgatata aatatcagaa agagattata ttcaaagaat 120

ga 122

<210> 39
<211> 122
<212> DNA
<213> Homo sapiens

<400> 39
cagcctcatt ctctcgtgtt tcttgctcat tgcacactct gtggcttcta ttgggggtat 60

cgtttctgag tctttaccac tgttcatcct tgaggggtca gttgaagtct ctccttgtct 120

gg 122

<210> 40
<211> 122
<212> DNA
<213> Homo sapiens

<400> 40
ctcatggaaa gagagggcag gatgagaggg agaataataa ctcacatact ctctcacaca 60

cgcaaacaca caaccagaga gctgctagag tagtcctgga ctgaggtcaa aatgtatggg 120

gt 122

<210> 41
<211> 122
<212> DNA
<213> Homo sapiens

<400> 41
aaaaaaaaaa gggtagtgac aggcagtaac ctcactatat gttaaaacat attacaaaca 60

cgcaacaaat taggccagta tgttaccagt gtaagaaaag caatggtaga tgataagttt 120

tt 122

<210> 42
<211> 122
<212> DNA
<213> Homo sapiens

<400> 42
tcaaaggagg aagggaaaga gactcgatct tgtaggagga aggacgagat gtgtgaaaat 60

cgtttactga ttttttattg atagaggccg tttttgcaat gctgactgcc caatcagcaa 120

gc 122

<210> 43
<211> 122
<212> DNA
<213> Homo sapiens

<400>  43
tgcgttgttt ccctctatga gtccatgtgt tctcatcatt tagcttctaa ctgttgcttt      60

cgtagtgtac ctctgcctca tctctcacga agtttgcagg tgagtttgag ccagggctga     120

gg                                                                    122


<210>  44
<211>  122
<212>  DNA
<213>  Homo sapiens

<400>  44
ctaatgggtg aaaaaaataa cccattaaca tactaggtgc tgattccaaa gcataaagta      60

cgataccact cagtatgaag ttcgagtgat tctttaaaaa ttgaacattt ccaaaaatgt     120

gt                                                                    122


<210>  45
<211>  122
<212>  DNA
<213>  Homo sapiens

<400>  45
gtaaaatcgc cgttttctag cctacaggca tttcccttgt cccgtttcaa caaacttcca      60

cggcccaatt tgttattcaa aaatttatgg aaaatgtatg ttaaagtgaa aagggtacat     120

tg                                                                    122


<210>  46
<211>  122
<212>  DNA
<213>  Homo sapiens

<400>  46
ggacagcccc acaacaagcc atgatccagc ccaaaatgtc aatcatgcca gttgagaaat      60

cgctattatc cacagaggaa ctgaggtaag caaaatcacc atttttcttt gaggatttag     120

gt                                                                    122


<210>  47
<211>  122
<212>  DNA
<213>  Homo sapiens

<400>  47
ccaagacagt cctcaattcc tctgttcaaa tccccagaga attctgacat gtaggaactt      60

cgtaagaagg tgcacaccag tgcctgccaa tgaaggttat aggtgacagg agagaggggg     120

ca                                                                    122


<210>  48
<211>  122
<212>  DNA

<213>  Homo sapiens

<400>  48
attagaactt cctaaggctg tgccacaagc tcatcctcaa ctttgacaaa ataaagtttc      60

cgaattgact gagacctgac tcagacactt ttgggatcat aatagcattc agccacttct     120

ca                                                                    122


<210>  49
<211>  122
<212>  DNA
<213>  Homo sapiens

<400>  49
tctcacatgg gtttctgggc taagatgaaa agtttggtgc aaaagccctc caagtagcaa      60

cgagaggtgt tagcatatca aggtgtcatg gtatgacatt ttcattccat cttaactttt     120

tt                                                                    122


<210>  50
<211>  122
<212>  DNA
<213>  Homo sapiens

<400>  50
gtcccagaat ccagagctag ggttgcttgc atagtgctta agggtagagt atacaaagaa      60

cgccaggaac ctgatgccaa gccctacagg gaactttcag gttataattc cagcaagccg     120

at                                                                    122


<210>  51
<211>  122
<212>  DNA
<213>  Homo sapiens

<400>  51
taaggaccaa aaatcacatg tctgtctttg tttccactaa gcctcaggag catgctgttg      60

cgtggtaaat atatacgagt atgtttgttc accaaacatt cccacagtat gggaagtgtt     120

tg                                                                    122


<210>  52
<211>  122
<212>  DNA
<213>  Homo sapiens

<400>  52
tcagctcatt cgtgctggga gaccatctct gagttagggt cacctctgca tggcaagtgt      60

cgtcgggaaa ttgtgggaac tggctggaga atgccaggct tctacttgtc ccagctaaat     120

ta                                                                    122


<210>  53

<211>  122
<212>  DNA
<213>  Homo sapiens

<400>  53
taatttttca ctacattata catctacctt ttagcccttc agactgaggc tatattttgc      60

cgttttgtga agataaaacc acagtgatga gagattgctg tagcttttca actccttgga      120

aa                                                                    122


<210>  54
<211>  122
<212>  DNA
<213>  Homo sapiens

<400>  54
accctaggag tgggcatgag ggctctgctg gaaccatgtg ccctaaagac aatgccgaag      60

cgtgcaactg aaaatgaaaa tgaaactgta acttgtgacc atctatctgt gtcttctttc      120

gc                                                                    122


<210>  55
<211>  122
<212>  DNA
<213>  Homo sapiens

<400>  55
gacaaagtag cagggtgggc gccgggggct gggagtgggg cacggggagc tcgtgttgaa      60

cggggaccga gtttccgttt gggaagatgg agagtcgcag acatgctgca cagtgtggat      120

gt                                                                    122


<210>  56
<211>  122
<212>  DNA
<213>  Homo sapiens

<400>  56
tcatagcttt aaagacactg ttgacatcct atggtgttgg ctacgtggag gagtcgggtc      60

cgagactgtg aggatgaggg ggtccaagtg ccatgtcctg ggagcagcta cctgggggct      120

ca                                                                    122


<210>  57
<211>  122
<212>  DNA
<213>  Homo sapiens

<400>  57
cccggcgcaa acacaacaaa gcccgcagac attctgtccc cacgaagaac ggacgccact      60

cgacttccta gcatcttgac gggctatcca agcgcggagt cagtttataa ggtgggtaac      120

ca                                                                    122

<210>  58
<211>  122
<212>  DNA
<213>  Homo sapiens

<400>  58
atccattatt ggctctgttt acccaaggga aaattaagtg cagggcctta gaacagtaag      60

cgctttaata cgcggatgca tatgctgatc tctgacagat gttggtcttt gggaacaaca     120

ga                                                                    122


<210>  59
<211>  122
<212>  DNA
<213>  Homo sapiens

<400>  59
gacacagaat gtcctgacct cagtggggtc tcaccacgac cctccactgt aggggggtcc      60

cgctgccacc ctccacgcta gaggggtctc accacgaccc tccaccctag acaggtcccg     120

cc                                                                    122


<210>  60
<211>  122
<212>  DNA
<213>  Homo sapiens

<400>  60
agaaagcaca atgtgaagaa aaagaggcga gaacgacccc cgaaccaacc aaagcccgcg      60

cgccgctgca tccggcgccc acgcccacgt cccactgccg tcgccaccgc cgccgagatc     120

at                                                                    122


<210>  61
<211>  122
<212>  DNA
<213>  Homo sapiens

<400>  61
tcttgtcgcg tgctgacgtc acatacgtgc ttatgcatag gcgagggcca catgcatcct      60

cgtttttgct ttctaaagca gcgcatcagg ccacacctct gagcttcctc ttgcagtttt     120

gg                                                                    122


<210>  62
<211>  122
<212>  DNA
<213>  Homo sapiens

<400>  62
atatatagtt gtaaatgtga tctcaaatat tattgagctg cagaatggat acagttctgc      60

cgtgtcccaa cttgtggttt ttaacagctg gggttcaaaa acttgtttta gattctgcat     120

ca                                                                  122

<210>  63
<211>  122
<212>  DNA
<213>  Homo sapiens

<400>  63
cagtcaaaag gaccggatgc cgtggagcag ggggcgatgc ccgttgggga ggctctggcc      60

cgtttgggga gcccactgtg ggggatcttg ggcatggcag gctgcatgtc ggagccttgc     120

cc                                                                  122

<210>  64
<211>  122
<212>  DNA
<213>  Homo sapiens

<400>  64
tgaaagaaaa aaatacacac tattttctag atcacaaaca gccccatcat tttaaattct      60

cgtaatgaac aacactggcc tctgctggta accaaggtcg tgatgaacca tccaggctgc     120

tg                                                                  122

<210>  65
<211>  122
<212>  DNA
<213>  Homo sapiens

<400>  65
atgggattca actgtggaaa ctgcaagttt ggcttttggg gaccaaactg cacagagaga      60

cgactcttgg tgagaagaaa catcttcgat ttgagtgccc cagagaagga caaatttttt     120

gc                                                                  122

<210>  66
<211>  122
<212>  DNA
<213>  Homo sapiens

<400>  66
gcggcagagg ccaaagtcta taaatttctg actctgcgcc ctactcattg tccgagtttc      60

cgcgtttttc atgaacgctc tcggccgccg ggaattctct ttgatgggat ttcaggtttc     120

ag                                                                  122

<210>  67
<211>  122
<212>  DNA
<213>  Homo sapiens

<400>  67
tcctgggggc ttctttccac gaagcctcac gtcgccggct gctctttcag ggcgccccca      60

cgtctgcttg accccgtagc ccatcacacg caccagcgtt ctttcattta ctcaaccaag 120

gg 122

<210> 68
<211> 122
<212> DNA
<213> Homo sapiens

<400> 68
tggatgcata gctgatttga ggtatctgca aaacaaatga tgggtcattt caacccatga 60

cgaatcttga agaatgatgg attgtcatat gagctaaaca gccaaggcta taccagaaac 120

ct 122

## Description

### Technical Field

The present invention relates to a method for determining prognosis of endometrial cancer.

### Background Art

Endometrial cancer is a cancer that develops in endometrium, and is one of gynecological malignancies most frequently found in developed countries. Although frequently found in postmenopausal women, cases of onset of endometrial cancer have been notably increasing in recent years among younger premenopausal women aged 40 or below. Current major therapy of endometrial cancer is surgery, that is, hysterectomy, bilateral salpingo-oophorectomy, and optional lymphoidectomy. Fertility preservation therapy is, however, chosen in some cases particularly for younger patients who wish pregnancy in the future, in order to avoid surgical menopause. Hormone therapy using medroxyprogesterone acetate (MPA) and the like is one known fertility preservation therapy. Such MPA therapy reportedly showed excellent therapeutic effect on early endometrial tumor including atypical hyperplasia and early stage endometrial cancer.

In other aspect, the fertility preservation therapy has been suffering from a problem of recurrence. It has been reported that recurrence rate of early stage endometrial cancer patient who underwent the MPA therapy reached approximately 63% (Non-Patent Literature 1), meaning that the MPA therapy suffers from higher risk of recurrence as compared with the surgical therapy. Moreover, the MPA therapy is effective mostly for early stage patients who are not observed to have invasion into myometrium. Hence, the MPA therapy, when applied, needs carful observation of the patients not only regarding their wishes to preserve fertility, but also compatibility of the patients to the therapy, considering the stage and risk of recurrence of cancer.

Abnormal methylation of DNA has been found to closely correlate with malignant transformation, and has therefore been attracting attention. A characteristic epigenetic abnormality in cancer cell has been known to relate to abnormal methylation of DNA in CpG island. The CpG island is a region where a two-base sequence of cytosine (C)-guanine (G), linked by a phosphodiester bond (p), appears with high frequency, and is often found in the promoter region upstream of a gene. Abnormal methylation of DNA in the CpG island relates to carcinogenesis, typically by way of inactivation of tumor suppressor gene. Non-Patent Literature 2 describes 13 genes which the methylation level is increased in CpG islands of, in correlation with increase of DNA methylation level in endometrial cancer patients. Non-Patent Literature 3 describes DNA methylation marker that can serve as a marker for high risk group of endometrial cancer. Non-Patent Literature 4 identifies approximately 27,000 and approximately 15,700 DNA regions that cause methylation respectively in relation to endometrioid cancer and serous cancer of uterine corpus, including some regions common to both cancers. Non-Patent Literature 5 identifies 12 regions that cause methylation in relation to ovarian cancer and endometrial cancer, including CpG island in ZNF154 promoter.

Methods based on bisulfite method have already been established as methods for analyzing methylated DNA, and have been widely used. Currently applied methods for analyzing methylated DNA, based on the bisulfite method, include Methylation-Specific PCR (MSP), Combined Bisulfite Restriction Analysis (COBRA), BAC-Array based Methylated CpG island Amplification (BAMCA), sequencing using next generation sequencer (MethylCap-seq), method for detecting a single-base extension reaction by using methylated/non-methylated DNA-specific probe (Infinium (registered trademark) assay), and methylation-sensitive restriction enzyme digestion sequencing (MRE-seq).

Patent Literature 1 discloses a method for detecting risk of poor prognosis of renal cell cancer by detecting methylation level at CpG sites in specific genes by, for example, bead array method, mass spectrometry (MassARRAY method), pyrosequencing, methylation-sensitive high resolution melting curve analysis, quantitative PCR, direct sequencing of bisulfite-treated product, and COBRA method. Patent Literature 2 discloses a method for determining prognosis of renal cell cancer, by detecting methylation level at CpG sites in specific genes, on the basis of difference of retention time in ion exchange chromatography.

### Citation List

### Patent Literature

Patent Literature 1: WO 2013/168644
Patent Literature 2: WO 2015/129916

### Non Patent Literature

Non Patent Literature 1: J. Gynecol. Oncol., 2018, 29:e21
Non Patent Literature 2: PLoS One, 2017, 12:e0173242
Non Patent Literature 3: Epigenetics, 2017, 12:19-26
Non Patent Literature 4: BMC Genomics, 2014, 15:868
Non Patent Literature 5: Epigenetics, 2013, 8:1355-72

### Summary of Invention

### Technical Problem

The present invention is to provide a method for determining status, prognosis or risk of onset of endometrial cancer on the basis of methylation level of DNA, and a method for acquiring data for the determination.

### Solution to Problem

The present inventors identified gene regions in endometrial cancer tissue, having methylation levels different from that in normal endometrial tissue, and further identified, from among these gene regions, those having methylation levels different depending on malignancy of endometrial cancer. The present inventors further found that prognosis of endometrial cancer, compatibility of endometrial cancer patients to preservation therapy, and risk of onset of endometrial cancer can be determined by measuring data regarding DNA methylation levels of such gene regions.

The present invention therefore provides the following:
[1] A method for detecting a cell or tissue derived from an endometrial cancer patient with poor prognosis, the method comprising:
   detecting DNA methylation level at a target CpG site in a genomic DNA derived from an endometrial cancer cell or tissue comprising the cell; and
   detecting a cell or tissue derived from the endometrial cancer patient with poor prognosis, on the basis of the detected DNA methylation level,
   wherein the target CpG site is at least one CpG site selected from the group consisting of CpG sites located at or near the chromosomal positions listed in Table A.
[2] A method for detecting a cell or tissue derived from an endometrial cancer patient compatible to preservation therapy, the method comprising:
   detecting DNA methylation level at a target CpG site in a genomic DNA derived from an endometrial cancer cell or tissue containing the cell; and
   detecting a cell or tissue derived from the endometrial cancer patient compatible to preservation therapy, on the basis of the detected DNA methylation level,
   wherein the target CpG site is at least one CpG site selected from the group consisting of CpG sites located at or near the chromosomal positions listed in Table A.
[3] A method for determining malignant transformation risk of endometrium, the method comprising:
   detecting DNA methylation level at a target CpG site in a genomic DNA derived from an endometrial cell or tissue containing the cell; and
   determining malignant transformation risk in the endometrium, on the basis of the detected DNA methylation level,
   wherein the target CpG site is at least one CpG site selected from the group consisting of CpG sites located at or near the chromosomal positions listed in Table A.
[4] A method for determining prognosis of endometrial cancer, the method comprising:
   detecting DNA methylation level at a target CpG site in a genomic DNA derived from an endometrial cancer cell or tissue containing the cell of a subject; and
   determining prognosis of endometrial cancer of the subject, on the basis of the detected DNA methylation level,
   wherein the target CpG site is at least one CpG site selected from the group consisting of CpG sites located at or near the chromosomal positions listed in Table A.
[5] A method for determining compatibility of an endometrial cancer patient to a preservation therapy, the method comprising:
   detecting DNA methylation level at a target CpG site in a genomic DNA derived from an endometrial cancer cell or tissue containing the cell of the endometrial cancer patient; and
   determining compatibility of the endometrial cancer patient to preservation therapy, on the basis of the detected DNA methylation level,
   wherein the target CpG site is at least one CpG site selected from the group consisting of CpG sites located at or near the chromosomal positions listed in Table A.
[6] A method for determining risk of endometrial cancer, the method comprising:
   detecting DNA methylation level at a target CpG site in a genomic DNA derived from an endometrial cell or tissue containing the cell of a subject; and
   determining risk of endometrial cancer of the subject, on the basis of the detected DNA methylation level,
   wherein the target CpG site is at least one CpG site selected from the group consisting of CpG sites located at or near the chromosomal positions listed in Table A.
[7] A method for acquiring data for use in detecting a cell or tissue derived from an endometrial cancer patient with poor prognosis, or in determining prognosis of endometrial cancer, the method comprising:
   detecting DNA methylation level at a target CpG site in a genomic DNA derived from an endometrial cancer cell or tissue containing the cell of a subject; and
   acquiring data for use in detecting a cell or tissue derived from the endometrial cancer patient with poor prognosis, or data for use in determining prognosis of endometrial cancer of the subject, on the basis of the detected DNA methylation level,
   wherein the target CpG site is at least one CpG site selected from the group consisting of CpG sites located at or near the chromosomal positions listed in Table A.
[8] A method for acquiring data for use in detecting a cell or tissue derived from an endometrial cancer patient compatible to a preservation therapy, or for use in determining compatibility of an endometrial cancer patient to the preservation therapy, the method comprising:
   detecting DNA methylation level at a target CpG site in a genomic DNA derived from an endometrial cancer cell or tissue containing the cell of the endometrial cancer patient; and
   acquiring data for use in detecting a cell or tissue derived from the endometrial cancer patient compatible to the preservation therapy, or data for use in determining compatibility of the endometrial cancer patient to the preservation therapy, on the basis of the detected DNA methylation level,
   wherein the target CpG site is at least one CpG site selected from the group consisting of CpG sites located at or near the chromosomal positions listed in Table A.
[9] A method for acquiring data for use in determining malignant transformation risk of endometrium, or for use in determining risk of endometrial cancer, the method comprising:
   detecting DNA methylation level at a target CpG site in a genomic DNA derived from an endometrial cell or tissue containing the cell of a subject; and
   acquiring data for use in determining malignant transformation risk of endometrium, or data for use in determining risk of endometrial cancer of the subject, on the basis of the detected DNA methylation level,
   wherein the target CpG site is at least one CpG site selected from the group consisting of CpG sites located at or near the chromosomal positions listed in Table A.
[10] The method according to any one of [1] to [9], wherein the target CpG site is at least one CpG site selected from the group consisting of CpG sites located at chromosome 10, position 106,400,259; chromosome 8, position 56,852,290; chromosome 14, position 60,973,823; chromosome 6, position 28,226,980; chromosome 1, position 62,660,624; chromosome 6, position 10,390,919; chromosome 8, position 132,321,917; chromosome 11, position 5,346,249; chromosome 21, position 31,972,506; chromosome 2, position 1,928,480; and chromosome 1, position 248,366,399, and CpG sites located near the positions.
[11] The method according to any one of [1] to [9], wherein the target CpG site is at least one CpG site selected from the group consisting of:
   CpG site contained in the DNA region of chromosome 10, from position 106,400,199 to position 106,400,320;
   CpG site contained in the region of chromosome 8, from position 56,852,230 to position 56,852,351;
   CpG site contained in the region of chromosome 14, from position 60,973,763 to position 60,973,884;
   CpG site contained in the region of chromosome 6, from position 28,226,920 to position 28,227,041;
   CpG site contained in the region of chromosome 1, from position 62,660,564 to position 62,660,685;
   CpG site contained in the region of chromosome 6, from position 10,390,859 to position 10,390,980;
   CpG site contained in the region of chromosome 8, from position 132,321,857 to position 132,321,978;
   CpG site contained in the region of chromosome 11, from position 5,346,189 to position 5,346,310;
   CpG site contained in the region of chromosome 21, from position 31,972,446 to position 31,972,567;
   CpG site contained in the region of chromosome 2, from position 1,928,420 to position 1,928,541; and
   CpG site contained in the region of chromosome 1, from position 248,366,339 to position 248,366,460.
[12] The method according to any one of [1] to [9], wherein the target CpG site is at least one CpG site selected from the group consisting of CpG sites contained in a DNA region consisting of any one of nucleotide sequences represented by SEQ ID NOs 1 to 11 or complementary sequences thereof.
[13] The method according to any one of [1] to [12], wherein the detection of the DNA methylation level comprises detecting the DNA methylation level at the target CpG site, by using the genomic DNA which has been treated with bisulfite.
[14] The method according to [13], wherein the DNA methylation level is detected by pyrosequencing, mass spectrometry, bead array method, or ion exchange chromatography.
[15] A primer or a probe for use in determining prognosis of endometrial cancer, endometrial cancer patient compatible to preservation therapy, or risk of endometrial cancer,
   wherein the primer or the probe has at least 12 base long, and is hybridizable with a target CpG site which has been treated with bisulfite, and
   the target CpG site is at least one CpG site selected from the group consisting of CpG sites located at or near the chromosomal positions listed in Table A.
[16] The primer or the probe according to [15], wherein the target CpG site is at least one CpG site selected from the group consisting of CpG sites located at chromosome 10, position 106,400,259; chromosome 8, position 56,852,290; chromosome 14, position 60,973,823; chromosome 6, position 28,226,980; chromosome 1, position 62,660,624; chromosome 6, position 10,390,919; chromosome 8, position 132,321,917; chromosome 11, position 5,346,249; chromosome 21, position 31,972,506; chromosome 2, position 1,928,480; and chromosome 1, position 248,366,399, and CpG sites located near the positions.
[17] The primer or the probe according to [15], wherein the target CpG site is at least one CpG site selected from the group consisting of the following CpG sites:
   CpG site contained in the region of chromosome 10, from position 106,400,199 to position 106,400,320;
   CpG site contained in the region of chromosome 8, from position 56,852,230 to position 56,852,351;
   CpG site contained in the region of chromosome 14, from position 60,973,763 to position 60,973,884;
   CpG site contained in the region of chromosome 6, from position 28,226,920 to position 28,227,041;
   CpG site contained in the region of chromosome 1, from position 62,660,564 to position 62,660,685;
   CpG site contained in the region of chromosome 6, from position 10,390,859 to position 10,390,980;
   CpG site contained in the region of chromosome 8, from position 132,321,857 to position 132,321,978;
   CpG site contained in the region of chromosome 11, from position 5,346,189 to position 5,346,310;
   CpG site contained in the region of chromosome 21, from position 31,972,446 to position 31,972,567;
   CpG site contained in the region of chromosome 2, from position 1,928,420 to position 1,928,541; and
   CpG site contained in the region of chromosome 1, from position 248,366,339 to position 248,366,460.
[18] The primer or the probe according to [15], wherein the target CpG site is at least one CpG site selected from the group consisting of CpG sites contained in a DNA region consisting of any one of nucleotide sequences represented by SEQ ID NOs 1 to 11 or complementary sequences thereof.

**[Table A]**

| CpG site | | | | | |
|---|---|---|---|---|---|
| Chromosome No. | Chromosomal position | Chromosome No. | Chromosomal position | Chromosome No. | Chromosomal position |
| 10 | 106400259 | 13 | 112759355 | 10 | 18583838 |
| 8 | 56852290 | 7 | 35301188 | 12 | 61961255 |
| 14 | 60973823 | 14 | 29254530 | 10 | 26461882 |
| 6 | 28226980 | 7 | 49815502 | 8 | 98376014 |
| 1 | 62660624 | 2 | 105459164 | 2 | 1984549 |
| 6 | 10390919 | 1 | 91185422 | 10 | 87364316 |
| 8 | 132321917 | 4 | 41749443 | 8 | 139114773 |
| 11 | 5346249 | 6 | 27647843 | 7 | 116151921 |
| 21 | 31972506 | 8 | 65549360 | 10 | 135030554 |
| 2 | 1928480 | 10 | 118138962 | 12 | 132102188 |
| 1 | 248366399 | 8 | 114435951 | 2 | 1417164 |
| 13 | 112711380 | 5 | 152981133 | 8 | 72872845 |
| 4 | 147561203 | 16 | 5293539 | 7 | 157933750 |
| 6 | 29943464 | 2 | 119418938 | 1 | 229706215 |
| 6 | 10391237 | 14 | 82292196 | 10 | 132461281 |
| 5 | 140787623 | 12 | 59657344 | 8 | 65528567 |
| 1 | 221050491 | 11 | 107067568 | 5 | 84126213 |
| 6 | 27649120 | 11 | 6049230 | 8 | 65525631 |
| 19 | 52452447 | 2 | 2321788 | 11 | 88911467 |
| 5 | 150326174 | 11 | 5295849 | 10 | 130844884 |
| 6 | 10393778 | 1 | 152797107 | 16 | 1088479 |
| 18 | 70535389 | 5 | 166491296 | 7 | 126746802 |
| 4 | 111550830 | 5 | 166988043 | | |

### Advantageous Effects of Invention

The present invention makes it possible to determine prognosis (including malignancy and risk of recurrence) of endometrial cancer, compatibility of endometrial cancer patients to preservation therapy, and risk of onset of endometrial cancer, with high sensitivity and specificity. The present invention enables early diagnosis of endometrial cancer. The present invention also provides a guideline for determining treatment policy for endometrial cancer patients, such as determine whether surgery or preservation therapy should be chosen. The present invention can provide information on benefit of preservation therapy, and risk of recurrence after preservation therapy, for younger endometrial cancer patients who wish future pregnancy, and to improve QOL and survival rate of the patients. The present invention can also provide data for use in determining or diagnosing these events.

### Brief Description of Drawings

Fig. 1 illustrates a hierarchical clustering of endometrial cancer patients (n = 74), on the basis of DNA methylation level at 63,033 CpG sites that demonstrate abnormal methylation in a group of endometrial cancer patients.
Fig. 2 illustrates a hierarchical clustering of juvenile endometrial cancer patients (n = 34), on the basis of DNA methylation level at 40,589 CpG sites that demonstrate abnormal methylation in a group of juvenile endometrial cancer patients.

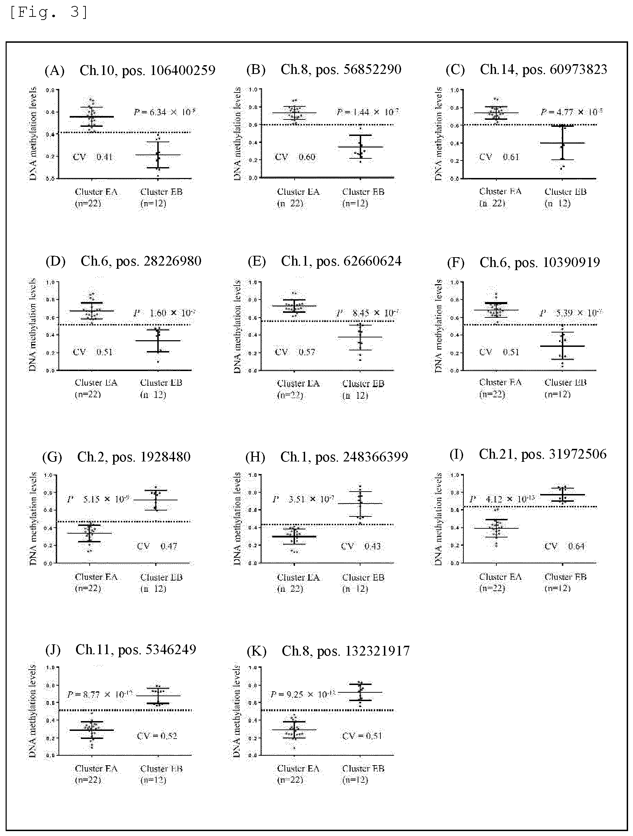

Fig. 3 illustrates a scattergram of methylation level at CpG sites, including (A) chromosome 10, position 106,400,259; (B) chromosome 8, position 56,852,290; (C) chromosome 14, position 60,973,823; (D) chromosome 6, position 28,226,980; (E) chromosome 1, position 62,660,624; (F) chromosome 6, position 10,390,919; (G) chromosome 2, position 1,928,480; (H) chromosome 1, position 248,366,399; (I) chromosome 21, position 31,972,506; (J) chromosome 11, position 5,346,249; and (K) chromosome 8, position 132,321,917. CV represents diagnostic threshold value (cutoff value) at which (YE-A) group and (YE-B) group is discriminated on an ROC curve. A p-value obtained from Welch's t test is given to each drawing.

### Description of Embodiments

"Endometrial cancer" in the present specification is referred to as cancer that develops in endometrium, and more specifically to endometrioid cancer. The "endometrial cancer" in the present specification includes primary cancer and recurrent cancer. "Juvenile endometrial cancer" in the present specification is referred to as endometrial cancer in premenopausal women, more specifically in women of reproductive age, and more specifically in women aged 40 or under. Meanwhile, "senile endometrial cancer" in the present specification is referred to as endometrial cancer other than juvenile endometrial cancer, and more specifically endometrial cancer in women aged over 40 years, or women over reproductive age.

"Risk of cancer" in the present specification is referred to as current onset of cancer, or risk of future onset. "Malignant transformation risk" of cell or tissue in the present specification means that the cell or tissue currently has cancer, or risk of having cancer in the future. "Risk of recurrence" of cancer in the present specification is referred to as current recurrence of cancer, or risk of future recurrence. "Risk of recurrence" of endometrial cancer in the present specification is specifically referred to as risk of recurrence of endometrial cancer after preservation therapy.

"Malignancy" in the present specification is referred to as stage or grade of cancer. The stage of endometrial cancer may be determined according to surgeical staging prepared by the International Federation of Gynecology and Obstetrics (FIGO), or tumor node metastasis (TNM) classification prepared by the Union for International Cancer Control (UICC). "High malignancy" cancer in the present specification is referred to as cancer at more advanced stage or at higher grade.

"Prognosis" of cancer in the present specification is referred to as future status of cancer. It is understood that, for example, the higher the possibility of improvement of cancer, the "better" (or non-poorer) the prognosis would be, whereas the lower the possibility of improvement, the "worse" (or poorer) the prognosis would be. "Prognosis" of cancer reflects malignancy and risk of recurrence of the cancers. It is understood that the higher the malignancy or risk of recurrence of cancer, the "worse" (or poorer) the prognosis would be. For example, poorer prognosis of cancer can mean that the cancer has higher malignancy or higher risk of recurrence, meanwhile better prognosis of cancer can mean that the cancer has lower malignancy or lower risk of recurrence.

"Preservation therapy" of endometrial cancer in the present specification is referred to as a method of cancer therapy that preserves uterus of the patient, and more specifically means fertility preservation therapy, that is, a method for treating cancer so as to preserve fertility of the patient. Examples of the preservation therapy include non-surgical treatments such as chemical therapy, radiation therapy, hormone therapy, and combinations of them. Examples of the hormone therapy include progesterone therapy by, for example, administration of medroxyprogesterone acetate (MPA). The preservation therapy may alternatively be any combination of the aforementioned non-surgical treatments and surgical treatment (such as partial endometrial resection), so long as fertility of the patient can be preserved. The "preservation therapy" in the present specification is more specifically referred to as progesterone therapy by, for example, administration of medroxyprogesterone acetate (MPA), or a combination of the progesterone therapy and surgical treatment that can preserve fertility of the patient.

"CpG site" in the present specification means a site where cytosine (C) and guanine (G) are linked by a phosphodiester bond (p) in DNA. A region where the CpG sites appear with high frequency is referred to as CpG island. The CpG island is often found at a position close to the coding region of gene, for example, in the promoter region, so that the CpG site of gene is often found in the CpG island that resides closer to the coding region of gene, or in the promoter region of gene. "CpG site of (a certain) gene" in the present specification, when not specifically defined, preferably means a CpG site contained in the CpG island that resides closer to the coding region of such gene, and more preferably means a CpG site that resides in the promoter region of such gene.

"DNA methylation" in the present specification means a status where the carbon atom at the 5-position of cytosine in DNA is methylated. "DNA methylation level" at a certain CpG site in the present specification means ratio of methylation of DNA at the CpG site. Highness and lowness of the DNA methylation level in the present specification respectively mean highness and lowness of the ratio of methylation of DNA.

One aspect of the present invention relates to a method for determining prognosis of endometrial cancer. An embodiment of this aspect relates to a method for determining prognosis of endometrial cancer, the method includes:
detecting DNA methylation level at a target CpG site in a genomic DNA derived from an endometrial cancer cell or tissue containing the cell of a subject; and
determining prognosis of endometrial cancer of the subject, on the basis of the detected DNA methylation level. Another embodiment of this aspect relates to a method for detecting a cell or tissue derived from an endometrial cancer patient with poor prognosis, the method includes:
detecting DNA methylation level at a target CpG site in a genomic DNA derived from an endometrial cancer cell or tissue containing the cell of a subject; and
detecting a cell or tissue derived from the endometrial cancer patient with poor prognosis, on the basis of the detected DNA methylation level. Examples of the subject in this aspect inlcude subjects in need of determination of prognosis of endometrial cancer, such as endometrial cancer patients including those before treatment, during treatment or after treatment of endometrial cancer. The endometrial cancer patient is preferably a juvenile endometrial cancer patient. Preferred example of the subject includes patients who currently, or wish to, undergo preservation therapy of endometrial cancer, and more preferred example includes juvenile endometrial cancer patients currently, or wish to, undergo fertility preservation therapy.

Another aspect of the present invention relates to a method for determining compatibility of an endometrial cancer patient to a preservation therapy. An embodiment of this aspect relates to a method for determining compatibility of an endometrial cancer patient to a preservation therapy, the method includes:
detecting DNA methylation level at a target CpG site in a genomic DNA derived from an endometrial cancer cell or tissue containing the cell of an endometrial cancer patient; and
determining compatibility of the endometrial cancer patient to the preservation therapy, on the basis of the detected DNA methylation level. Another embodiment of this aspect relates to a method for detecting a cell or tissue derived from an endometrial cancer patient compatible to a preservation therapy, the method includes:
   detecting DNA methylation level at a target CpG site in a genomic DNA derived from an endometrial cancer cell or tissue containing the cell of an endometrial cancer patient; and
   detecting a cell or tissue derived from the endometrial cancer patient compatible to the preservation therapy, on the basis of the detected DNA methylation level. Examples of the endometrial cancer patient in this aspect include patients in need of determination of compatibility to the preservation therapy. Preferred example of the endometrial cancer patient includes patients who currently, or wish to, undergo preservation therapy of endometrial cancer, and more preferred example includes juvenile endometrial cancer patients who currently, or wish to, undergo fertility preservation therapy.

Still another aspect of the present invention relates to a method for determining risk of endometrial cancer. An embodiment of this aspect relates to a method for determining risk of endometrial cancer in a subject, the method includes:
detecting DNA methylation level at a target CpG site in a genomic DNA derived from an endometrial cell or tissue containing the cell of a subject; and
determining risk of endometrial cancer of the subject, on the basis of the detected DNA methylation level. Another embodiment of this aspect relates to a method for determining malignant transformation risk of endometrium, the method includes:
detecting DNA methylation level at a target CpG site in a genomic DNA derived from an endometrial cell or tissue containing the cell of a subject; and
determining malignant transformation risk of endometrium of the subject, on the basis of the detected DNA methylation level. Examples of the subject in this aspect include subjects in need of determination of risk of endometrial cancer, such as medical examinees and suspected patients of endometrial cancer.

Examples of the genomic DNA derived from an endometrial cell, endometrial cancer cell, or tissue containing them, used in the method of the present invention include genomic DNA prepared from endometrial cell, endometrial cancer cells, or tissue containing the cell of the subject. Examples of the endometrial cell, endometrial cancer cell or tissue containing the cell include a fresh endometrial cell, endometrial cancer cell or tissue containing the cell, which are sampled by surgery or biopsy; a frozen endometrial cell, frozen endometrial cancer cell or frozen tissue containing the cell, which are frozen after sampled; and an endometrial cell, endometrial cancer cell or tissue containing the cell, which are formalin-fixed and embedded in paraffin after sampled. Among them, a frozen endometrial cell, frozen endometrial cancer cell or tissue containing the cell are preferred, from the viewpoint of suppressing decomposition of the genomic DNA in the cell or the tissue, and of detecting the DNA methylation level more efficiently.

The technique for preparing a sample DNA from the tissue or the cell may suitably be selected from among known methods, without special limitation. Examples of known methods for preparing DNA are exemplified by phenolchloroform method, and DNA extraction methods with use of commercially available DNA extraction kit such as QIAamp DNA Mini kit (from Qiagen), Clean Columns (from Nextec Inc.), AquaPure (from Bio-Rad Laboratories, Inc.), ZR Plant/Seed DNA Kit (from Zymo Research), prepGEM (from ZyGEM Co., Ltd.), and BuccalQuick (from TrimGen Corp.).

The prepared genomic DNA is preferably treated with bisulfite. The method for treating DNA with bisulfite may suitably be selected from among known methods, without special limitation. Examples of known methods for the bisulfite treatment include methods with use of commercially available kit such as EZ DNA Methylation-Gold^{™} kit (from Zymo Research), EpiTect Bisulfite Kit (48) (from Qiagen), MethylEasy (from Human Genetic Signatures Pty), Cells-to-CpG Bisulfite Conversion Kit (from Applied Biosystems), and CpGenome Turbo Bisulfite Modification Kit (from MERCK MILLIPORE).

The bisulfite-treated DNA is further preferably amplified. The method of amplification is preferably PCR, although not specifically limited. The method and condition for amplification may suitably be selected from known methods and conditions, depending on sequence, length and quantity of DNA to be amplified.

As detailed later in Examples, the present inventors found that the DNA methylation level at the CpG sites listed in Tables 1 to 3 are different between a patient group with high malignancy of endometrial cancer, and a patient group with low malignancy of endometrial cancer. In particular, the DNA methylation level at the CpG sites listed in Table 1 made it possible, in Examples descried later, to discriminate the patients with high malignancy and the patients with low malignancy, with 100% sensitivity and specificity. The DNA methylation level at the CpG sites listed in Tables 1 to 3 reflect progression (malignancy) of endometrial cancer, and can therefore be used as a marker that represents prognosis (including malignancy and risk of recurrence) and risk of endometrial cancer.

**[Table 1]**

| | | | | |
|---|---|---|---|---|
| (A) | | | | |

| CpG site | | | | Change of methylation with progress of cancer |
|---|---|---|---|---|
| Chromosome No. | Chromosomal position | Gene name | NCBI Gene ID | |
| 10 | 106400259 | (Non-coding) | - | Increase |
| 8 | 56852290 | LYN | 4067 | |
| 14 | 60973823 | (Non-coding) | - | |
| 6 | 28226980 | ZKSCAN4 | 387032 | |
| 1 | 62660624 | L1TD1 | 54596 | |
| 6 | 10390919 | (Non-coding) | - | |
| (B) | | | | |

| CpG site | | | | Change of methylation with progress of cancer |
|---|---|---|---|---|
| Chromosome No. | Chromosomal position | Gene name | NCBI Gene ID | |
| 8 | 132321917 | (Non-coding) | - | Decrease |
| 11 | 5346249 | (Non-coding) | - | |
| 21 | 31972506 | (Non-coding) | - | |
| 2 | 1928480 | MYT1L | 23040 | |
| 1 | 248366399 | OR2M3 | 127062 | |

**[Table 2]**

| CpG site | | | Change of methylation with progress of cancer |
|---|---|---|---|
| Chromosome No. | Chromosomal position | Gene name | |
| 13 | 112711380 | SOX1-OT | Increase |
| 4 | 147561203 | POU4F2 | |
| 6 | 29943464 | HCG9 | |
| 6 | 10391237 | (Non-coding) | |
| 5 | 140787623 | PCDHGA9 | |
| | | PCDHGB5 | |
| | | PCDHGA8 | |
| | | PCDHGB4 | |
| | | PCDHGA7 | |
| | | PCDHGA6 | |
| | | PCDHGB3 | |
| | | PCDHGA5 | |
| | | PCDHGB2 | |
| | | PCDHGA4 | |
| | | PCDHGB1 | |
| | | PCDHGA3 | |
| | | PCDHGA2 | |
| | | PCDHGA1 | |
| 1 | 221050491 | HLX-AS1 | |
| 6 | 27649120 | (Non-coding) | |
| 19 | 52452447 | ZNF350-AS1 | |
| 5 | 150326174 | ZNF300P1 | |
| 6 | 10393778 | (Non-coding) | |
| 18 | 70535389 | (Non-coding) | |
| 4 | 111550830 | PITX2 | |
| 13 | 112759355 | SOX1-OT | |
| 7 | 35301188 | (Non-coding) | |
| 14 | 29254530 | LINC02282 | |
| 7 | 49815502 | VWC2 | |
| 2 | 105459164 | LINC01158 | |
| 1 | 91185422 | (Non-coding) | |
| 4 | 41749443 | PHOX2B | |
| 6 | 27647843 | -(Non-coding) | |

**[Table 3]**

| CpG site | | | Change of methylation with progress of cancer |
|---|---|---|---|
| Chromosome No. | Chromosomal position | Gene name | |
| 8 | 65549360 | CYP7B1 | Decrease |
| 10 | 118138962 | CCDC172 | |
| 8 | 114435951 | CSMD3 | |
| 5 | 152981133 | GRIA1 | |
| 16 | 5293539 | (Non-coding) | |
| 2 | 119418938 | (Non-coding) | |
| 14 | 82292196 | (Non-coding) | |
| 12 | 59657344 | (Non-coding) | |
| 11 | 107067568 | (Non-coding) | |
| 11 | 6049230 | (Non-coding) | |
| 2 | 2321788 | MYT1L | |
| 11 | 5295849 | (Non-coding) | |
| 1 | 152797107 | (Non-coding) | |
| 5 | 166491296 | (Non-coding) | |
| 5 | 166988043 | TENM2 | |
| 10 | 18583838 | CACNB2 | |
| 12 | 61961255 | (Non-coding) | |
| 10 | 26461882 | MYO3A | |
| 8 | 98376014 | LOC101927066 | |
| 2 | 1984549 | MYT1L | |
| 10 | 87364316 | GRID1-AS1 | |
| 8 | 139114773 | (Non-coding) | |
| 7 | 116151921 | (Non-coding) | |
| 10 | 135030554 | KNDC1 | |
| 12 | 132102188 | (Non-coding) | |
| 2 | 1417164 | TPO | |
| 8 | 72872845 | MSC-AS1 | |
| 7 | 157933750 | PTPRN2 | |
| 1 | 229706215 | (Non-coding) | |
| 10 | 132461281 | (Non-coding) | |
| 8 | 65528567 | CYP7B1 | |
| 5 | 84126213 | (Non-coding) | |
| 8 | 65525631 | CYP7B1 | |
| 11 | 88911467 | TYR | |
| 10 | 130844884 | (Non-coding) | |
| 16 | 1088479 | (Non-coding) | |
| 7 | 126746802 | GRM8 | |

Hence, examples of the target CpG sites for which the DNA methylation level is detected in the method of the present invention include CpG sites located at or near the chromosomal positions listed in Tables 1 to 3. The method of the present invention may therefore only detect the DNA methylation level for at least one CpG site selected from the group consisting of the CpG sites located at or near the chromosomal positions listed in Tables 1 to 3.

In the present specification, the chromosomal positions of the CpG sites are denoted, on the basis of positions on the NCBI Database Genome Reference Consortium Human Build 37 (GRCh37), which is a reference human genomic sequence. In the present specification, gene names are based on those registered in the NCBI database ([www.ncbi.nlm.nih.gov/gene]) .

"Near" the chromosomal position in the present specification means a region ranges from 500-bases upstream (5'-side of DNA) to 500-bases downstream (3'-side of DNA), preferably from 200-bases upstream to 200-bases downstream, more preferably 100-bases upstream to 100-bases downstream, and even more preferably 60-bases upstream to 60-bases downstream on both sides of such chromosomal position.

Examples of the CpG sites located at or near the chromosomal positions listed in Tables 1, 2 and 3 respectively include CpG sites contained in the DNA regions listed in Tables 4, 5 and 6.

**[Table 4]**

| DNA Region | | |
|---|---|---|
| Chromosome No. | Chromosomal region | Sequence |
| 10 | Positions 106,400,199 - 106,400,320 | (SEQ.ID.NO 1) |
| 8 | Positions 56,852,230 - 56,852,351 | (SEQ ID NO 2) |
| 14 | Positions 60,973,763 - 60,973,884 | (SEQ ID NO 3) |
| 6 | Positions 28,226,920 - 28,227,041 | (SEQ ID NO 4) |
| 1 | Positions 62,660,564 - 62,660,685 | (SEQ ID NO 5) |
| 6 | Positions 10,390,859 - 10,390,980 | (SEQ ID NO 6) |
| 8 | Positions 132,321,857 - 132,321,978 ; | (SEQ ID NO 7) |
| 11 | Positions 5,346,189 - 5,346,310 | (SEQ ID NO 8) |
| 21 | Positions 31,972,446 - 31,972,567 | (SEQ ID NO 9) |
| 2 | Positions 1,928,420 - 1,928,541 | (SEQ ID NO 10) |
| 1 | Positions 248,366,339 - 248,366,460 | (SEQ ID NO 11) |

**[Table 5]**

| DNA Region | | |
|---|---|---|
| Chromosome No. | Chromosomal region | Sequence |
| 13 | Positions 112,711,320 - 112,711,441 | (SEQ ID NO 12) |
| 4 | Positions 147,561,143 - 147,561,264 | (SEQ ID NO 13) |
| 6 | Positions 29,943,404 - 29,943,525 | (SEQ ID NO 14) |
| 6 | Positions 10,391,177 - 10,391,298 | (SEQ ID NO 15) |
| 5 | Positions 140,787,563 - 140,787,684 | (SEQ ID NO 16) |
| 1 | Positions 221,050,431 - 221,050,552 ; | (SEQ ID NO 17) |
| 6 | Positions 27,649,060 - 27,649,181 | (SEQ ID NO 18) |
| 19 | Positions 52,452,387 - 52,452,508 | (SEQ ID NO 19) |
| 5 | Positions 150,326,114 - 150,326,235 | (SEQ ID NO 20) |
| 6 | Positions 10,393,718 - 10,393,839 | (SEQ ID NO 21) |
| 18 | Positions 70,535,329 - 70,535,450 | (SEQ ID NO 22) |
| 4 | Positions 111,550,770 - 111,550,891 | (SEQ ID NO 23) |
| 13 | Positions 112,759,295 - 112,759,416 ; | (SEQ ID NO 24) |
| 7 | Positions 35,301,128 - 35,301,249 | (SEQ ID NO 25) |
| 14 | Positions 29,254,470 - 29,254,591 | (SEQ ID NO 26) |
| 7 | Positions 49,815,442 - 49,815,563 | (SEQ ID NO 27) |
| 2 | Positions 105,459,104 - 105,459,225 | (SEQ ID NO 28) |
| 1 | Positions 91,185,362 - 91,185,483 | (SEQ ID NO 29) |
| 4 | Positions 41,749,383 - 41,749,504 | (SEQ ID NO 30) |
| 6 | Positions 27,647,783 - 27,647,904 | (SEQ ID NO 31) |

**[Table 6]**

| DNA Region | | |
|---|---|---|
| Chromosome No. | Chromosomal region | Sequence |
| 8 | Positions 65,549,300 - 65,549,421 | (SEQ ID NO 32) |
| 10 | Positions 118,138,902 - 118,139,023 | (SEQ ID NO 33) |
| 8 | Positions 114,435,891 - 114,436,012 | (SEQ ID NO 34) |
| 5 | Positions 152,981,073 - 152,981,194 | (SEQ ID NO 35) |
| 16 | Positions 5,293,479 - 5,293,600 | (SEQ ID NO 36) |
| 2 | Positions 119,418,878 - 119,418,999 ; | (SEQ ID NO 37) |
| 14 | Positions 82,292,136 - 82,292,257 | (SEQ ID NO 38) |
| 12 | Positions 59,657,284 - 59,657,405 | (SEQ ID NO 39) |
| 11 | Positions 107,067,508 - 107,067,629 | (SEQ ID NO 40) |
| 11 | Positions 6,049,170 - 6,049,291 | (SEQ ID NO 41) |
| 2 | Positions 2,321,728 - 2,321,849 | (SEQ ID NO 42) |
| 11 | Positions 5,295,789 - 5,295,910 | (SEQ ID NO 43) |
| 1 | Positions 152,797,047 - 152,797,168 | (SEQ ID NO 44) |
| 5 | Positions 166,491,236 - 166,491,357 | (SEQ ID NO 45) |
| 5 | Positions 166,987,983 - 166,988,104 | (SEQ ID NO 46) |
| 10 | Positions 18,583,778 - 18,583,899 | (SEQ ID NO 47) |
| 12 | Position 61,961,195 - 61,961,316 | (SEQ ID NO 48) |
| 10 | Positions 26,461,822 - 26,461,943 | (SEQ ID NO 49) |
| 8 | Positions 98,375,954 - 98,376,075 | (SEQ ID NO 50) |
| 2 | Positions 1,984,489 - 1,984,610 | (SEQ ID NO 51) |
| 10 | Positions 87,364,256 - 87,364,377 | (SEQ ID NO 52) |
| 8 | Positions 139,114,713 - 139,114,834 | (SEQ ID NO 53) |
| 7 | Positions 116,151,861 - 116,151,982 | (SEQ ID NO 54) |
| 10 | Positions 135,030,494 - 135,030,615 | (SEQ ID NO 55) |
| 12 | Positions 132,102,128 - 132,102,249 | (SEQ ID NO 56) |
| 2 | Positions 1,417,104 - 1,417,225 | (SEQ ID NO 57) |
| 8 | Positions 72,872,785 - 72,872,906 | (SEQ ID NO 58) |
| 7 | Positions 157,933,690 - 157,933,811 | (SEQ ID NO 59) |
| 1 | Positions 229,706,155 - 229,706,276 | (SEQ ID NO 60) |
| 10 | Positions 132,461,221 - 132,461,342 | (SEQ ID NO 61) |
| 8 | Positions 65,528,507 - 65,528,628 | (SEQ ID NO 62) |
| 5 | Positions 84,126,153 - 84,126,274 | (SEQ ID NO 63) |
| 8 | Positions 65,525,571 - 65,525,692 | (SEQ ID NO 64) |
| 11 | Positions 88,911,407 - 88,911,528 | (SEQ ID NO 65) |
| 10 | Positions 130,844,824 - 130,844,945 | (SEQ ID NO 66) |
| 16 | Positions 1,088,419 - 1,088,540 | (SEQ ID NO 67) |
| 7 | Positions 126,746,742 - 126,746,863 | (SEQ ID NO 68) |

In other words, examples of the target CpG sites for which the DNA methylation level is detected include CpG sites contained in the DNA regions consisting of any nucleotide sequences represented by SEQ ID NOs 1 to 68 or complementary sequences thereof.

In the method of the present invention, it suffices to detect the DNA methylation level for at least one CpG site from among the aforementioned target CpG sites. For example in the method of the present invention, it suffices to detect the DNA methylation level for at least one CpG site selected from the group consisting of the CpG sites contained in the DNA regions listed in Tables 4, 5 and 6. In the present specification, it alternatively suffices to detect the DNA methylation level for at least one CpG site selected from the group consisting of CpG sites contained in the DNA regions consisting of nucleotide sequences represented by SEQ ID NOs 1 to 68 or complementary sequences thereof.

The target CpG sites used in the method of the present invention is preferably at least one CpG site selected from the group consisting of CpG sites located at chromosome 10, position 106,400,259; chromosome 8, position 56,852,290; chromosome 14, position 60,973,823; chromosome 6, position 28,226,980; chromosome 1, position 62,660,624; chromosome 6, position 10,390,919; chromosome 8, position 132,321,917; chromosome 11, position 5,346,249; chromosome 21, position 31,972,506; chromosome 2, position 1,928,480; and chromosome 1, position 248,366,399, and CpG sites located near the positions.

In a preferred embodiment, the target CpG site used in the method of the present invention is at least one CpG site selected from the group consisting of:
CpG site contained in the region of chromosome 10, from position 106,400,199 to position 106,400,320;
CpG site contained in the region of chromosome 8, position 56,852,230 to position 56,852,351;
CpG site contained in the region of chromosome 14, from position 60,973,763 to position 60,973,884;
CpG site contained in the region of chromosome 6, from position 28,226,920 to position 28,227,041;
CpG site contained in the region of chromosome 1, from position 62,660,564 to position 62,660,685;
CpG site contained in the region of chromosome 6, from position 10,390,859 to position 10,390,980;
CpG site contained in the region of chromosome 8, from position 132,321,857 to position 132,321,978;
CpG site contained in the region of chromosome 11, from position 5,346,189 to position 5,346,310;
CpG site contained in the region of chromosome 21, from position 31,972,446 to position 31,972,567;
CpG site contained in the region of chromosome 2, from position 1,928,420 to position 1,928,541; and
CpG site contained in the region of chromosome 1, from position 248,366,339 to position 248,366,460.

In a preferred embodiment, the target CpG site used in the method of the present invention is at least one CpG site selected from the group consisting of CpG sites contained in the DNA regions consisting of nucleotide sequences represented by SEQ ID NOs 1 to 11 or complementary sequences thereof.

In a more preferred embodiment, the target CpG sites for which the DNA methylation level is detected in the method of the present invention is preferably at least one CpG site selected from the group consisting of CpG sites located at chromosome 10, position 106,400,259; chromosome 8, position 56,852,290; chromosome 14, position 60,973,823; chromosome 6, position 28,226,980; chromosome 1, position 62,660,624; chromosome 6, position 10,390,919; chromosome 8, position 132,321,917; chromosome 11, position 5,346,249; chromosome 21, position 31,972,506; chromosome 2, position 1,928,480; and chromosome 1, position 248,366,399.

The aforementioned CpG sites may be combined for use as the target, from the viewpoint of improving sensitivity or specificity in detection of the DNA methylation level. Examples of the combination include a combination of any one of CpG sites listed in Table 1 with a CpG site located near them; a combination of any two or more of the CpG sites listed in Table 1; a combination of all CpG sites listed in Table 1; and a combination of any one or more CpG sites listed in Table 1 with any one or more CpG sites listed in Table 2 or 3. Among them, a combination of any one of CpG sites listed in Table 1 with a CpG site near them; a combination of any two or more of the CpG sites listed in Table 1; and a combination of all CpG sites listed in Table 1 are preferred.

Hence in a case where the bisulfite-treated DNA is amplified by, for example, PCR in the method of the present invention as described above, the DNA region containing the target CpG site is amplified. Preferably, the DNA region containing the target CpG site is amplified.

More preferably, in a case where the bisulfite-treated DNA is amplified by, for example, PCR in the method of the present invention, a part of, or the entirety of any of the DNA regions listed in Tables 4 to 6 is amplified. Even more preferably, a part of, or the entirety of any of the DNA regions listed in Table 4 is amplified. Size of the DNA region to be amplified is not specifically limited, so long as the target CpG site is contained therein.

In the method of the present invention, a technique for detection of the DNA methylation level at the CpG site may only be a technique capable of quantifying the DNA methylation level at the given CpG site, and may suitably be selected from known techniques. Examples of such known techniques include first to eight techniques enumerated below.

The first technique is a method of quantifying the DNA methylation at the CpG site by using a single base extension reaction with a probe prepared to have a base complementary to methylated cytosine or non-methylated cytosine at the 3'-terminal. The first technique is, for example, based on the principle below. First, the genomic DNA is subjected to the bisulfite treatment. The bisulfite treatment converts the non-methylated cytosine residue into uracil, whereas the methylated cytosine residue remains unconverted (see Clark SJ, et al., Nucleic Acids Res., 1994, vol. 22, p. 2990-7). Next, whole genome amplification is conducted using the thus bisulfite-treated genomic DNA as a template, the product is then fragmented by using an enzyme (typically fragmented into approximately 300 to 600 bp), and allowed to dissociate into a single strand.

In the first technique, a probe which is hybridizable with the genomic DNA converted by the bisulfite treatment, and in which the base at the 3'-terminal is a base complementary to cytosine in the CpG site is prepared. That is, when such CpG site is methylated, the 3'-terminal base of the probe is guanine, whereas when such CpG site remains unmethylated, the 3' terminal base of the probe is adenine.

Such two kinds of probes different from each other only in the 3'-terminal base complementary to the CpG site, are then hybridized with the aforementioned single-strand DNA fragment, and the product is subjected to the single base extension reaction in the presence of a fluorescence-labeled base. As a consequence, in a case where the CpG site in the single-strand fragment is methylated, the probe having guanine as the 3'-terminal base (methylation detecting probe) has the fluorescence-labeled base incorporated by the single base extension reaction, whereas the probe having adenine as the 3'-terminal base (non-methylation detecting probe) does not cause the single base extension reaction due to mismatching of the 3'-terminal base, and does not have the fluorescence-labeled base incorporated therein. On the other hand, in a case where the CpG site in the single-strand fragment is not methylated, the non-methylation detecting probe has the fluorescence-labeled base incorporated therein, whereas the methylation detecting probe does not have the fluorescence-labeled base incorporated therein. The DNA methylation level may therefore be calculated from intensity of fluorescence emitted from the methylation detecting probe and/or the non-methylation detecting probe.

In another aspect, the first technique may employ a probe hybridizable with the genomic DNA converted by the bisulfite treatment, in which the 3'-terminal base is a base complementary to guanine in the CpG site, in place of employing the aforementioned methylation detecting probe or the non-methylation detecting probe. Such probe is then hybridized with the aforementioned single-strand DNA fragment, and the product is subjected to the single base extension reaction in the presence of guanine labeled with a fluorescent substance, and/or adenine labeled with a fluorescent dye different from the fluorescent substance. As a consequence, in a case where the CpG site is methylated, the probe has fluorescence-labeled guanine incorporated therein, whereas in a case where the CpG site is not methylated, the probe has fluorescence-labeled adenine incorporated therein, so that the DNA methylation level can therefore be calculated from intensity of fluorescence emitted from the individual fluorescent substances incorporated into the probes.

Examples of such first technique preferably include bead array method (Infinium (registered trademark) assay, for example).

The second technique is a method of quantifying the methylated DNA by mass spectrometry. The second technique is, for example, based on the principle below. First, the genomic DNA is subjected to the bisulfite treatment. Next, a DNA containing at least one of the aforementioned CpG sites is amplified with a T7 promoter-added primer using the bisulfite-treated genomic DNA as a template. Next, the amplified DNA is transcribed into RNA, and then subjected to a base-specific cleavage reaction with RNase. The cleavage reaction products are then analyzed by using a mass spectrometer for mass spectrometry.

Mass of the product derived from methylated cytosine residue (mass of cytosine) and mass of the product derived from non-methylated cytosine residue (mass of uracil), obtained by the mass spectrometry, are compared to calculate the DNA methylation level at the CpG site.

Preferred examples of such second technique include DNA methylation analysis with a mass spectrometer (MassARRAY (registered trademark), see Jurinke C, et al., Mutat. Res., 2005, 573, p.83-95, and Patent Literature 2, for example).

MassARRAY (registered trademark) amplifies the bisulfite-treated DNA that contains the CpG site, transcribes it into RNA, and specifically cleaves the product at uracil with an RNAase. This produces RNA fragments having different lengths depending on presence or absence of DNA methylation. The obtained RNA fragments are subjected to mass spectrometry, and thereby the fragments having methylated CpG site and the non-methylated fragments are separately detected according to difference of molecular weight. The primer used for amplifying the DNA containing the CpG site may be designed by using, for example, EpiDesigner (software for designing primer for use in MassARRAY, from SEQUENOM). Mass spectrometry of the RNA fragments employs a MALDI-TOF MAS (for example, MassARRAY Analyzer 4 from SEQUENOM), capable of detecting a mass difference of a single base. The DNA methylation level is calculated from a mass ratio of the RNA fragment derived from methylated DNA and the RNA fragment derived from non-methylated DNA.

The third technique is based on the principle below. First, the genomic DNA is subjected to the bisulfite treatment. Although the non-methylated cytosine residue is converted to uracil by the bisulfite treatment, uracil is represented by thymine in the extension reaction (sequencing reaction) below. Next, a DNA containing at least one of the aforementioned CpG sites is amplified, using the bisulfite-treated genomic DNA as a template. The amplified DNA is then dissociated into single strands. Next, one strand is isolated from the thus dissociated single strand DNAs. The strand is then extended base by base, from the vicinity of a base in the CpG site, during which pyrophosphoric acid produced therein is enzymatically allowed to cause luminescence, and luminescence intensity is measured. Luminescence intensity ascribed to methylated cytosine residues (luminescence intensity of cytosine) and luminescence intensity ascribed to non-methylated cytosine residues (luminescence intensity of thymine) are compared, and the DNA methylation level (%) at the CpG site is then calculated by, for example, using the equation below.

DNA methylation level (%) = luminescence intensity of cytosine × 100/(luminescence intensity of cytosine + luminescence intensity of thymine).

The third technique is, for example, Pyrosequencing (registered trademark) (see Anal. Biochem., (2000)10:103-110) .

The fourth technique is, for example, based on the principle below. First, the genomic DNA is subjected to the bisulfite treatment. Next, in a reaction system containing an intercalator that causes fluorescence upon being intercalated into the double strand of DNA, a nucleotide that contains at least one CpG site is amplified using the bisulfite-treated genomic DNA as a template. Next, temperature of the reaction system is changed, and change of fluorescence intensity of the intercalator is detected. A melting curve of the nucleotide containing at least one CpG site is compared with a melting curve of a product amplified by using a methylated or non-methylated control sample as a template, and the DNA methylation level at the CpG site is calculated.

Examples of the fourth technique include methylation-sensitive high resolution melting curve analysis (MS-HRM) (see Wojdacz TK, et al., Nat. Protoc., 2008, 3, p. 1903-8).

The fifth technique is based on the principle below. First, the genomic DNA is subjected to the bisulfite treatment. Next, a primer set that enables amplification when the CpG site is methylated, and a primer set that enables amplification when the CpG site is not methylated are prepared. Next, a nucleotide containing at least one of the aforementioned CpG sites is amplified by using each of these primer sets and the bisulfite-treated genomic DNA as a template. The amount of the thus obtained amplified product, that is, the amount of amplified product specific to the methylated CpG site and the amount of amplified product specific to the non-methylated CpG site are compared, to thereby calculate the DNA methylation level at the CpG site.

In another aspect of the fifth technique, first the genomic DNA is subjected to the bisulfite treatment. Next, an oligonucleotide probe that contains a nucleotide hybridizable when the CpG site is methylated and that is labeled with a reporter fluorescent dye and a quencher fluorescent dye is prepared. Next, an oligonucleotide probe that contains a nucleotide hybridizable when the CpG site is not methylated and that is labeled with a reporter fluorescent dye different from the aforementioned reporter fluorescent dye, and with the quencher fluorescent dye is also prepared. The bisulfite-treated genomic DNA is then hybridized with each of the oligonucleotide probes, and a nucleotide that contains the CpG site is then amplified using, as a template, such genomic DNA hybridized with the oligonucleotide probe. Fluorescence emitted from the reporter fluorescent dye, upon decomposition of the oligonucleotide probe during amplification, is then detected. Intensity of the thus detected fluorescence emitted from the reporter fluorescent dye which is specific to the methylated cytosine CpG site is compared with intensity of fluorescence emitted from the reporter fluorescent dye which is specific to the non-methylated cytosine CpG site, to thereby calculate the DNA methylation level at the CpG site.

Examples of the fifth technique include methylation-specific polymerase chain reaction (MS-PCR) using real-time quantitative PCR, such as MethyLight method usingTaqMan probe (registered trademark).

The sixth technique is based on the principle below. First, the genomic DNA is subjected to the bisulfite treatment. Next, a direct sequencing reaction is allowed to proceed using, as a template, a nucleotide that contains the bisulfite-converted CpG site. Fluorescence intensity ascribed to a determined base sequence, that is, fluorescence intensity ascribed to the methylated cytosine residue (fluorescence intensity of cytosine) is then compared with fluorescence intensity ascribed to the non-methylated cytosine residue (luminescence intensity of thymine), to thereby calculate the DNA methylation level at the CpG site.

In another aspect of the sixth technique, first the genomic DNA is subjected to the bisulfite treatment. Next, a nucleotide that contains the bisulfite-treated CpG site is cloned by, for example, a PCR reaction. Base sequences of the thus obtained individual cloned products are then determined, and the number of the cloned products having the base sequence specific to the methylated cytosine CpG site is compared with the number of the cloned products having the base sequence specific to the non-methylated cytosine CpG site, to thereby calculate the DNA methylation level at the CpG site.

Examples of the sixth technique include bisulfite direct sequencing, and bisulfite cloning sequencing (see Kristensen LS, et al., Clin. Chem., 2009, 55, p. 1471-83).

The seventh technique is based on the principle below. First, the genomic DNA is subjected to the bisulfite treatment. Next, a region that contains the CpG site is amplified by PCR using, as a template, a nucleotide that contains the bisulfite-treated CpG site. The amplified DNA fragment is then treated with a restriction enzyme that can recognize a site having different sequences depending on whether the CpG site is methylated or not methylated. Restriction enzyme fragment derived from the methylated CpG site and restriction enzyme fragment derived from the non-methylated CpG site are then fractionated by electrophoresis, and the band density is quantitatively analyzed to thereby calculate the DNA methylation level at the CpG site.

Examples of the seventh technique include combined bisulfite restriction analysis (COBRA).

The eighth technique is a method using ion exchange chromatography. The eighth technique is based on the principle below. First, the genomic DNA is subjected to the bisulfite treatment. The product is then fragmented to obtain a DNA fragment that contains the CpG site. A region containing the CpG site is then amplified by PCR using the obtained DNA fragment as a template. The amplified DNA fragment is then subjected to ion exchange chromatography, to thereby separate DNA having the methylated CpG site and DNA having the non-methylated CpG site.

Chain length of the PCR-amplified product in the eighth technique may suitably be selected in view of factors including shortening of PCR amplification time, shortening of analysis time by ion exchange chromatography, and keeping of separation performance. The chain length of the PCR-amplified product, in case of using a CpG site-rich sample DNA, is preferably 1000 bp or shorter, more preferably 700 bp or shorter, and even more preferably 500 bp or shorter. Meanwhile, the chain length of the PCR-amplified product, in case of using a CpG site-poor sample DNA, is 30 to 40 bp at the shortest, which is the chain length of PCR-amplified product in case of using a primer of approximately 15 mer long at which non-specific hybridization in PCR is avoidable. On the other hand, the primer is preferably designed so as to enrich the CpG site. For example, the content of cytosine at the CpG site preferably accounts for 2% or more, more preferably 5% or more relative to the chain length of the PCR-amplified product.

According to the eighth technique, the non-methylated cytosine residue is converted into uracil during the bisulfite treatment of the genomic DNA, and then converted into thymine by PCR. On the other hand, the methylated cytosine residue remains unchanged even after the bisulfite treatment and PCR. Due to such difference of the bases, a fragment that contains methylated cytosine (methylated fragment) and a non-methylated fragment are detectable as separate peaks with different retention times in ion exchange chromatography. More specifically, the methylated fragment is detectable as a peak whose retention time is shorter than that of the non-methylated fragment. Whether DNA at the CpG site is methylated or not can therefore be determined on the basis of the retention time of the peaks in ion exchange chromatography. In a case where a plurality of CpG sites are contained in DNA to be subjected to ion exchange chromatography, the more the CpG sites are methylated, the shorter the retention time of the peak is. The DNA methylation level at the CpG site can therefore be calculated on the basis of the retention time of the peak. It may alternatively be possible to calculate abundance or abundance ratio of each of the methylated fragment and the non-methylated fragment, on the basis of area or height of the peaks.

Whether the DNA at the CpG site is methylated or not, or, the DNA methylation level at the CpG site is preferably determined by comparing the DNA methylation level at the CpG site with a known sample (control), or by using a calibration curve preliminarily prepared by using samples with known DNA methylation levels. Still alternatively, one can preliminarily determine, by using samples with known DNA methylation levels, a retention time at which the retention time of peak of the methylated fragment having highly methylated CpG site is discriminable from the retention time of peak of the fragment having a low methylation level (also referred to as reference retention time in the present specification). For example, any fragment detected earlier than the reference retention time is determined to be highly-methylated DNA.

Ion exchange chromatography employed in the eighth technique is preferably anion exchange chromatography. Column filler may only be a base particle having a strong cationic group on the surface thereof, and is preferably a base particle having both of strong cationic group and a weak cationic group on the surface thereof, as described in WO 2012/108516. The base particle more preferably includes a coated polymer particle that contains a hydrophobic crosslinked polymer particle and a hydrophilic polymer layer containing the strong cationic group (preferably quaternary ammonium salt) and copolymerized on the surface of the hydrophobic crosslinked polymer particle, and the weak cationic group (preferably tertiary amino group) introduced on the surface of the coated polymer particle. Column temperature during the chromatographic analysis is preferably 30°C or higher, and lower than 90°C.

The techniques suitably applicable to the "techniques for detecting DNA methylation level" in the present invention have been described, to which the techniques are however not limited. In the aforementioned first to eighth techniques, the genomic DNA prepared from an endometrial cell, endometrial cancer cell or tissue containing the cell are further subjected to the bisulfite treatment as described above. Hence in the method of the present invention, the genomic DNA used for detecting the DNA methylation level at the CpG site is preferably a bisulfite-treated genomic DNA derived from an endometrial cell, endometrial cancer cell, or tissue containing the cell.

According to the method of the present invention, prognosis of endometrial cancer (including malignancy and risk of recurrence), compatibility of a subject to the preservation therapy, or risk of endometrial cancer (malignant transformation risk of endometrium) can be determined on the basis of detected DNA methylation level at the CpG site.

In the determination of prognosis of endometrial cancer of the present invention, whether the prognosis of endometrial cancer of the subject is good or poor; whether or not endometrial cancer of the subject is high malignancy; or whether or not the risk of recurrence of endometrial cancer of the subject is high, is determined. Also, preferably, whether or not the cell or tissue used for detecting the DNA methylation level is derived from an endometrial cancer patient with poor prognosis, or from an endometrial cancer patient with high malignancy or high risk of recurrence is determined.

In the determination of compatibility of the subject to the preservation therapy of the present invention, preferably whether or not the subject is compatible to the preservation therapy of endometrial cancer. Also, preferably, whether or not the cell or tissue used for detecting the DNA methylation level is derived from the patient compatible to the preservation therapy of endometrial cancer is determined.

In the determination of risk of endometrial cancer of the present invention, preferably whether or not the endometrium of the subject has high malignant transformation risk, or whether or not the subject has high risk of endometrial cancer. More preferably, whether or not the endometrium of the subject has high risk of having endometrial cancer in the future, or whether or not the subject has high risk of onset of endometrial cancer in the future is determined. Alternatively, whether or not the cell or tissue used for detecting the DNA methylation level is derived from the subject with high risk of endometrial cancer is determined.

A skilled person in the art may suitably set specific indices (threshold values) for the aforementioned determination, depending on the techniques for detecting the DNA methylation level. Embodiments of determination procedures will be described below.

In a first embodiment for the determination, first, receiver operating characteristic (ROC) analysis is performed on each of the CpG sites for each of the techniques for detecting the DNA methylation level, to determine sensitivity (positive rate) and specificity

(negative rate). The DNA methylation level at which the sum of sensitivity and specificity reaches maximum may be set as a cutoff value. The cutoff value may be set on, for example, the basis of Youden's index.

In the first embodiment, for the CpG site where the methylation level is increased with progress of endometrial cancer (for example, CpG sites at or near the chromosomal positions listed in Table 1(A) and Table 2), when the DNA methylation level detected by the method of the present invention is higher than the cutoff value, the DNA methylation level is determined to exceed a diagnostic threshold value, and the subject is then determined to belong to a poor prognosis group of endometrial cancer or belong to a high risk group of endometrial cancer, or not to belong to a compatible group to preservation therapy. In contrast, when the detected methylation level is equal to or below the cutoff value, the subject is then determined not to belong to the poor prognosis group of endometrial cancer, or not to belong to the high risk group of endometrial cancer, or to belong to the compatible group to preservation therapy.

On the other hand, for the CpG site where the methylation level is decreased with progress of endometrial cancer (for example, CpG sites at or near the chromosomal positions listed in Table 1(B) and Table 3), when the DNA methylation level detected by the method of the present invention is lower than the cutoff value, the DNA methylation level is determined to exceed a diagnostic threshold value, and the subject is then determined to belong to the poor prognosis group of endometrial cancer, or to belong to the high risk group of endometrial cancer, or not to belong to the compatible group to preservation therapy. In contrast, when the detected methylation level is equal to or higher than the cutoff value, the subject is then determined not to belong to the poor prognosis group of endometrial cancer, or not to belong to the high risk group of endometrial cancer, or to belong to the compatible group to preservation therapy.

In a second embodiment for the determination, retention time of a peak obtained by ion exchange chromatographic analysis (the eighth technique) on DNA that contains the target CpG site derived from a subject (sample) is compared with retention time of DNA that contains the non-methylated target CpG site (negative control) or DNA that contains the methylated target CpG site (positive control), to thereby determine the methylation level of the CpG site, prognosis of cancer regarding endometrial cancer, risk, or compatibility to the preservation therapy.

In the second embodiment, for the CpG site where the methylation level is increased with progress of endometrial cancer (for example, CpG sites at or near the chromosomal positions listed in Table 1(A) and Table 2), when a peak is detected at a predetermined retention time shorter than that of a peak of the negative control from the sample, the DNA methylation level is determined to exceed a diagnostic threshold value, and the subject is then determined to belong to the poor prognosis group of endometrial cancer, or belong to the high risk group of endometrial cancer, or not to belong to the compatible group to preservation therapy. Preferably, when a peak is detected at a retention time equivalent to that of the positive control from the sample, the DNA methylation level is determined to exceed a diagnostic threshold value, and the subject is then determined to belong to the poor prognosis group of endometrial cancer, or to belong to the high risk group of endometrial cancer, or not to belong to the compatible group to preservation therapy. In contrast, when the DNA methylation level does not exceed the diagnostic threshold value, the subject is then determined not to belong to the poor prognosis group of endometrial cancer, or not to belong to the high risk group of endometrial cancer, or to belong to the compatible group to preservation therapy.

On the other hand, for the CpG site where the methylation level is decreased with progress of endometrial cancer (for example, CpG sites at or near the chromosomal positions listed in Table 1(B) and Table 3), when a peak is detected at a predetermined retention time longer than that of a peak of the positive control from the sample, the DNA methylation level is determined to exceed a diagnostic threshold value, and the subject is then determined to belong to the poor prognosis group of endometrial cancer, or to belong to the high risk group of endometrial cancer, or not to belong to the compatible group for preservation therapy. Alternatively, when a peak is detected at a retention time equivalent to that of the negative control from the sample, the DNA methylation level is determined to exceed a diagnostic threshold value, and the subject is then determined to belong to the poor prognosis group of endometrial cancer, or to belong to a high risk group of endometrial cancer, or not to belong to a compatible group to preservation therapy. In contrast, when the DNA methylation level does not exceed the diagnostic threshold value, the subject is then determined not to belong to the poor prognosis group of endometrial cancer, or not to belong to the high risk group of endometrial cancer, or to belong to the compatible group to preservation therapy.

In a case where the methylation level is detected at a plurality of CpG sites in the first and second embodiments, the number or ratio of the CpG sites at which the DNA methylation level exceeds the diagnostic threshold value may be used as an index for determination. For example, the subject may be determined to belong to the poor prognosis group of endometrial cancer, or to belong to the high risk group of endometrial cancer, or not to belong to the compatible group for preservation therapy, when the methylation level exceeds the diagnostic threshold value at all of the examined CpG sites. Alternatively, the subject may be determined to belong to the poor prognosis group of endometrial cancer, or to belong to the high risk group of endometrial cancer, or not to belong to the compatible group to preservation therapy, when the methylation level exceeds the diagnostic threshold value at a certain percentage or more of the examined CpG sites. Still alternatively, the subject may be determined to belong to the poor prognosis group of endometrial cancer, or to belong to the high risk group of endometrial cancer, or not to belong to the compatible group to preservation therapy, when the methylation level exceeds the diagnostic threshold value at a certain number of the examined CpG sites. In contrast, any subject not satisfying these criteria can be determined not to belong to the poor prognosis group of endometrial cancer, or not to belong to the high risk group of endometrial cancer, or to belong to the compatible group to preservation therapy.

As described above, the present invention can determine risk and prognosis of endometrial cancer. When early detection of the patients of high risk group regarding onset of endometrial cancer is enabled by the method of the present invention, it can provide preventive intervention to avoid onset of endometrial cancer, and can provide early treatment to improve vital prognosis of the patient. In particular, discrimination of endometrial cancer with low malignancy or small possibility of recurrence by the method of the present invention makes it possible to predict effectiveness of the preservation therapy to the patient. Prediction of effectiveness of the preservation therapy is especially beneficial to the patient of juvenile endometrial cancer who may become pregnant in the future. Although fertility preservation therapy such as MPA therapy is an important choice of therapy for a patient who wishes to become pregnant, it is effective only for early cancer patients, and suffers from higher risk of recurrence as compared with surgery. Hence, the preservation therapy, when applied, needs to be closely examined regarding compatibility of the patient to the therapy. The present invention can provide the patient who wishes the preservation therapy, with information on effectiveness of the preservation therapy to such patient, thereby contributing to improve QOL and vital prognosis of the patient.

The present invention therefore also relates to preventive intervention to the subject classified into the high risk group of endometrial cancer, using the method of risk determination of the present invention. This aspect of the present invention enables prevention, early diagnosis and early treatment of endometrial cancer in the subject of the high risk group.

The present invention also provides a therapeutic method for endometrial cancer, including treating the subject determined to have endometrial cancer by the method of determing risk of the present invention. The present invention still also provides a therapeutic method for endometrial cancer, including treating the subject classified into the poor prognosis group of endometrial cancer by the method of determining prognosis of the present invention. These aspects of the present invention enable to provide the subject having endometrial cancer, with suitable therapy further earlier. Examples of means for therapy include, but not limited to, surgery, chemical therapy, radiation therapy and hormone therapy such as MPA therapy.

In still further aspect, the present invention relates to provision of data for use in determination of the aforementioned risk and prognosis of endometrial cancer, or determination of compatibility of the patient to the preservation therapy. An embodiment of this aspect relates to a method for acquiring data for use in detecting a cell or tissue derived from an endometrial cancer patient with poor prognosis, or for determining prognosis of endometrial cancer, the method includes:
detecting DNA methylation level at a target CpG site in a genomic DNA derived from an endometrial cancer cell or tissue containing the cell of a subject; and
acquiring data for use in detecting a cell or tissue derived from the endometrial cancer patient with poor prognosis, or data for use in determining prognosis of endometrial cancer of the subject, on the basis of the detected DNA methylation level. Another embodiment of this aspect relates to a method for acquiring data for use in detecting a cell or tissue derived from an endometrial cancer patient compatible to a preservation therapy, or for use in determining compatibility of an endometrial cancer patient to the preservation therapy, the method includes:
   detecting DNA methylation level at a target CpG site in a genomic DNA derived from an endometrial cancer cell or tissue containing the cell of the endometrial cancer patient; and
   acquiring data for use in detecting a cell or tissue derived from the endometrial cancer patient compatible to the preservation therapy, or data for use in determining compatibility of the endometrial cancer patient to the preservation therapy, on the basis of the detected DNA methylation level. A still another embodiment of this aspect relates to a method for acquiring data for use in determining malignant transformation risk of endometrium, or for use in determining risk of endometrial cancer, the method includes:
   detecting DNA methylation level at a target CpG site in a genomic DNA derived from an endometrial cell or tissue containing the cell of a subject; and
   acquiring data for use in determining malignant transfomration risk of endometrium, or data for use in determining risk of endometrial cancer of the subject, on the basis of the detected DNA methylation level. Examples of the subject, procedures for preparing the genomic DNA and for detecting the DNA methylation level, and the target CpG sites at which the methylation level needs to be detected are same as those described above. As descried previously, the DNA methylation level at the CpG sites listed in Tables 1 to 3 can be used as a marker that represents prognosis and risk of endometrial cancer or compatibility to the preservation therapy.

The method for acquiring data of the present invention is a method that does not include diagnoses by any doctor or other persons on prognosis and risk of endometrial cancer or compatibility to the preservation therapy. For example, the method is conducted at, for example, research laboratories, clinical inspection laboratories and so forth, in order to acquire data necessary for diagnoses given by doctors or other persons.

The present invention also provides a primer or a probe for use in determining prognosis of endometrial cancer (including malignancy and risk of recurrence), determining compatibility of the subject to the preservation therapy of endometrial cancer, or determining risk of endometrial cancer (malignant transformation risk of endometrium). The primer or the probe has at least 12 base long, and is hybridizable with a target CpG site which has been treated with bisulfite.

An example of the primer or the probe of the present invention is a primer or a probe hybridizable with a CpG site contained in any of the DNA regions listed in Tables 4 to 6 (for example, a DNA region consisting of any of the nucleotide sequences represented by SEQ ID NOs 1 to 68, or a complementary sequence thereof). The primer or the probe is preferably is a primer or a probe hybridizable with a CpG site contained in any of the DNA regions listed in Table 4 (for example, a DNA region consisting of any of the nucleotide sequences represented by SEQ ID NOs 1 to 11, or a complementary sequence thereof).

A preferred example of the primer or the probe of the present invention is a primer or a probe consisting of any of the nucleotide sequences represented by SEQ ID NOs 1 to 68, or a complementary sequence thereof. Another preferred example of the primer or the probe of the present invention is a primer or a probe that has 95% or larger identity to any of the nucleotide sequences represented by SEQ ID NOs 1 to 68, or to a complementary sequence thereof, and can hybridize with the same CpG site as the CpG site with which the sequence can hybridize. Also, a fragment of the primer or the probe hybridizable with the same CpG site as the CpG site with which the primer or the probe can hybridize, can be used as the primer or the probe of the present invention.

More preferred example of the primer or the probe of the present invention is a primer or a probe consisting of any of the nucleotide sequences represented by SEQ ID NOs 1 to 11, or a complementary sequence thereof. Another further preferred example of the primer or the probe of the present invention is a primer or a probe that has 95% or larger identity to any of the nucleotide sequences represented by SEQ ID NOs 1 to 11, or to a complementary sequence thereof, and can hybridize with the same CpG site as the CpG site with which the sequence can hybridize. Also, a fragment of the primer or the probe hybridizable with the same CpG site as the CpG site with which the primer or the probe can hybridize, can be used as the primer or the probe of the present invention.

Another preferred example of the primer or the probe of the present invention is a primer or a probe that is hybridizable with a DNA fragment containing the target CpG site which has been treated with bisulfite (for example, a fragment that contains any one of nucleotide sequences represented by SEQ ID NOs 1 to 68, or a complementary strand thereof), and is constructed to have, at the 3'-terminal, a base complementary to methylated cytosine or non-methylated cytosine (for example, the primer or the probe applicable to the aforementioned first technique).

Another preferred example of the primer or the probe of the present invention is a primer (sequencing primer) that can cause base-by-base extension reaction from the vicinity of a base in the target CpG site which has been treated with bisulfite (for example, the primer or the probe applicable to the aforementioned third technique). Another preferred example is a probe hybridizable with a nucleotide that contains the target CpG site which has been treated with bisulfite (for example, the primer or the probe applicable to the aforementioned fourth technique).

Another preferred example of the primer or the probe of the present invention is a primer set that can specifically amplify a region that contains the methylated or non-methylated target CpG site, in a DNA fragment containing the target CpG site (for example, a fragment that contains any one of nucleotide sequences represented by SEQ ID NOs 1 to 68, or a complementary strand thereof) which has been treated with bisulfite (for example, the primer set applicable to PCR amplification in the aforementioned fifth technique or the eighth technique).

The primer or the probe of the present invention may only be at least 12 base long, preferably at least 15 base long, more preferably at least 20 base long, and even more preferably 15 to 200 base long. In case of the probe, it is preferably 100 to 200 base long, and more preferably 100 to 150 base long. In case of the PCR primer, it is preferably 12 to 60 base long, and more preferably 15 to 40 base long. The primer or the probe of the present invention may be labeled (fluorescent-labeled, for example). The primer or the probe of the present invention is preferably a primer or a probe applicable to any one of the aforementioned first to eighth techniques.

The present invention also provides a kit for use in determining prognosis of endometrial cancer, determining compatibility of the subject to the preservation therapy of endometrial cancer, or determining risk of endometrial cancer, which contains the primer or the probe of the present invention. The kit of the present invention is preferably used for determination of prognosis of endometrial cancer, determination of compatibility of the subject to the preservation therapy of endometrial cancer, determination of risk of endometrial cancer, or acquisition of data for use in determination of these events, according to any one of the aforementioned first to eighth techniques.

The kit of the present invention may contain components other than the primer or the probe of the present invention. Examples of such components include, but not limited to, reagents necessary for the bisulfite treatment (for example, a sodium bisulfite solutioin, etc.); reagents necessary for the PCR reaction (for example, deoxyribonucleotide, heat-resistant DNA polymerase, etc.); reagents necessary for Infinium (registered trademark) assay (for example, nucleotide labeled with fluorescent substance); reagents necessary for MassARRAY (registered trademark) (for example, RNase for use in base-specific cleavage); reagents necessary for pyrosequencing (for example, ATP sulfurylase for use in detecting pyrophosphoric acid, adenosine-5'-phosphosulfate, luciferase, luciferin, streptavidin for separating single-strand DNA, etc.); reagents necessary for MS-HRM (for example, intercalator that causes fluorescence when intercalated in double-strand DNA, etc.); and reagents necessary for detecting the label (for example, substrate and enzyme, positive control and negative control samples, buffer for use in dilution or washing of samples (tissue-derived genomic DNA, etc.), etc.). The kit may include the user's manual.

### Examples

The present invention will be detailed below referring to Examples, to which the present invention is not limited.

### [Patients and Tissue Samples]

The Example was conducted with the approval of the Ethics Committee of Keio University Hospital, in compliance with the Declaration of Helsinki. The tissue samples were obtained from the patients who underwent surgery at Keio University Hospital. Written informed consent was obtained from all patients.

Endometrial cancer tissue samples (n = 74) were obtained from group (E) of endometrial cancer patients with endometrioid cancer (aged 23 to 77). Endometrial tissue samples (n = 31) were obtained from group (C) of patients without endometrial cancer (aged 33 to 55). In group (E) of the endometrial cancer patients, 34 patients aged 40 or under were classified into juvenile endometrial cancer patient group (YE), and 40 patients aged over 40 years were classified into senile endometrial cancer patient group (OE) .

Histological diagnosis and grade diagnosis of cancer for group (E) were conducted according to WHO classification (2003) and TNM classification. Clinical stage was diagnosed according to FIGO Staging. Recurrence of cancer was diagnosed by a clinician on the basis of medical examination and image diagnosis. Clinicopathological factors for group (E) are listed in Table 7.

**[Table 7]**

| Clinicopathological factor | | Juvenile endometrioid cancer (YE) (age ≤ 40, n = 34) | Senile endometrioid cancer (OE) (age > 40, n = 40) |
|---|---|---|---|
| | | Average [Min. - Max.] | |
| Age | | 36[23-40] | 55 [42-77] |
| BMI | | 21.9[14.8-36.5] | 22.5[12.7-32.0] |
| | | Case (%) | |
| FIGO stage (2008) | | | |
| | I-II | 27 (79) | 35 (78) |
| | III-IV | 7 (21) | 5 (22) |
| Grade | | | |
| | G1-2 | 28 (82) | 35 (88) |
| | G3 | 6(18) | 5 (12) |
| Muscle invasion | | | |
| | < 1/2 | 29 (85) | 24 (60) |
| | ≥ 1/2 | 5 (15) | 16 (40) |
| Vascular invasion | | | |
| | No | 22 (65) | 22 (55) |
| | Yes | 12 (35) | 18 (45) |
| Lymph node metastasis | | | |
| | No | 29 (85) | 36 (90) |
| | Yes | 5 (15) | 4 (10) |
| Recurrence | | 1 (3) | 3 (8) |
| Fatal | | 00 | 1 (3) |

Sampled tissues were freeze-preserved. Each of the obtained fresh frozen tissue samples was treated with phenol-chloroform, and then dialyzed to extract a genomic DNA. Five hundred nanograms of the extracted DNA was then subjected to the bisulfite treatment, by using EZ DNA Methylation-Gold^{™} kit (from Zymo Research).

### [Example 1: Changes of DNA Methylation with Progress of Endometrial Cancer]

### (Detection of DNA Methylation Level by Infinium (Registered Trademark) Assay)

DNA methylation status at 866,895 CpG sites was analyzed by using Infinium (registered trademark) MethylationEPIC BeadChip (from Illumina, Inc.), at a resolution of 1 CpG site. According to a specification available from Illumina, Inc., the seller, objects to be analyzed by MethylationEPIC BeadChip include promoter region, 5' untranslated region, first exon, gene body and 3' untranslated region, and covers 99% of RefSeq genes and 96% of CpG islands.

The bisulfite-treated DNA was subjected to whole genome amplification, by using Infinium (registered trademark) assay kit (from Illumina, Inc.). The amplified DNA fragment was hybridized with a probe on the chip, and the hybridized DNA was then subjected to the single base extension reaction so as to incorporate a fluorescent-labeled base therein. In this way, each of the methylation detecting probe hybridized with the DNA fragment containing the methylated CpG and the non-methylation detecting probe hybridized with a DNA fragment containing the non-methylated CpG was fluorescent-labeled. Next, fluorescent signal was measured by using iScan Reader (from Illumina, Inc.), according to the manufacturer's protocol. The obtained data was analyzed by using GenomeStudio methylation software (from Illumina, Inc.). For each CpG site, a relative ratio of signal from the methylation detecting probe, to the total of signals from the methylation detecting probe and the non-methylation detecting probe was calculated. That is, the methylation level obtained from each CpG site was represented by so-called β value (range: 0.00 to 1.00).

### (Statistical Analysis)

The obtained data of methylation level was subjected to Welch's t test and Bonferroni correction, to thereby detect the CpG sites that demonstrate significant difference of methylation level between group (C) and group (E) (p < 0.01, and | group E average - group C average | ≥ 0.25). As a consequence, 63,033 CpG sites, demonstrating abnormal methylation in group (E) as compared with group (C), were identified.

[Example 2: Changes of DNA Methylation with Progress of Juvenile Endometrial Cancer]

The juvenile endometrial cancer patient group (YE) was subjected to Infinium (registered trademark) assay according to the same procedures as in Example 1, to thereby detect the CpG sites where the DNA methylation level changes with progress of cancer. As a consequence, 40,589 CpG sites, demonstrating abnormal methylation in group (YE) as compared with group (C), were identified.

[Example 3: Clustering of Whole Endometrial Cancer Based on DNA Methylation Profile]

(Hierarchical Clustering)

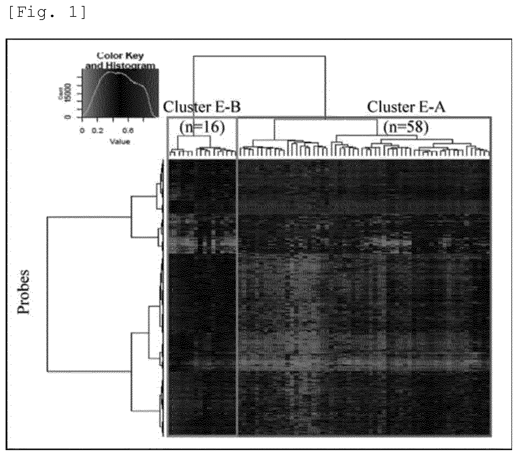

Hierarchical clustering (Euclidean distance, Ward's method, Canberra distance, complete linkage method) was carried for 63,033 CpG sites found in Example 1 on the basis of the DNA methylation level, to thereby classify group (E) into cluster A (group (E-A), n = 58) and cluster B (group (E-B), n = 16) (Fig. 1). Clinicopathological factors for group (E-A) and group (E-B) are listed in Table 8. Group (E-A) was found to be significantly older, found to cause vascular invasion significantly with more frequency, and found to show higher tendency of causing high grade G3 cases, as compared with group (E-B). All recurrent cases and fatal cases were included in group (E-A). Group (E-A) was therefore considered to be a patient group with high clinicopathological malignancy and poor prognosis.

**[Table 8]**

| Clinicopathological factor | | E-A (n = 58) | E-B (n = 16) | p-Value |
|---|---|---|---|---|
| | | Average [Min. - Max.] | | |
| Age | | 49[27-77] | 38[23-50] | 9.00×10⁻³ |
| BMI | | 21.6[12.7-36.5] | 24.1[14.8-31.2] | 8.40×10⁻¹ |
| | | Case (%) | | |
| FIGO stage (2008) | | | | |
| | I-II | 47 (81) | 15 (94) | 4.42×10⁻¹ |
| | III-IV | 11 (19) | 1 (6) | |
| Grade | | | | |
| | G1-2 | 47 (81) | 16 (100) | 1.06×10⁻¹ |
| | G3 | 11 (19) | 00 | |
| Muscle invasion | | | | |
| | < 1/2 | 40(69) | 13 (81) | 5.32×10⁻¹ |
| | ≥ 1/2 | 18 (31) | 3 (19) | |
| Vascular invasion | | | | |
| | No | 30 (52) | 14 (87) | 1.00×10⁻² |
| | Yes | 28 (48) | 2 (13) | |
| Lymph node metastasis | | | | |
| | No | 50 (86) | 15 (94) | 6.73×10⁻¹ |
| | Yes | 8 (14) | 1 (6) | |
| Recurrence | | 4(7) | 0 (0) | 5.70×10⁻¹ |
| Fatal | | 1 (2) | 0 (0) | 1.00 |

[Example 4: Clustering of Juvenile Endometrial Cancer Based on DNA Methylation Profile]

### (Hierarchical Clustering)

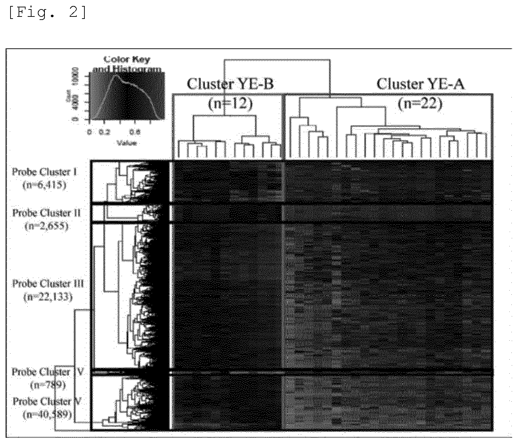

Hierarchical clustering was carried according to the same procedures as in Example 3 on the basis of the DNA methylation level, for 40,589 CpG sites found in Example 2, to thereby classify group (YE) into cluster A (group (YE-A), n = 22) and cluster B (group (YE-B), n = 12) (Fig. 2). Clinicopathological factors for group (YE-A) and group (YE-B) are listed in Table 9. Group (YE-A) was found to show higher tendencies of causing vascular invasion and high grade G3 cases, as compared with group (YE-B). Only one recurrent case was found in group (YE-A). Moreover, all patients in group (YE-A) were found to be included in group (E-A), and all patients in group (YE-B) were found to be included in group (E-B). From these results, group (YE-A) was considered to be a patient group with high clinicopathological malignancy and poor prognosis.

**[Table 9]**

| Clinicopathological factor | | YE-A (n = 22) | YE-B (n = 12) | p-Value |
|---|---|---|---|---|
| | | Average [Min. - Max.] | | |
| Age | | 36 [27-40] | 38 [23-40] | 3.17×10⁻¹ |
| BMI | | 21.0[16.2-36.5] | 24.1[14.8-31.2] | 6.63×10⁻¹ |
| | | Case (%) | | |
| FIGO stage (2008) | | | | |
| | I-II | 16 (73) | 11 (92) | 3.78×10⁻¹ |
| | III-IV | 6 (27) | 1 (8) | |
| Grade | | | | |
| | G1-2 | 16 (73) | 12 (100) | 6.91×10⁻² |
| | G3 | 6 (27) | 00 | |
| Muscle invasion | | | | |
| | < 1/2 | 19(86) | 10(83) | 1.00 |
| | ≥ 1/2 | 3(14) | 2 (17) | |
| Vascular invasion | | | | |
| | No | 12 (55) | 10 (83) | 1.40×10⁻¹ |
| | Yes | 10 (45) | 2 (17) | |
| Lymph node metastasis | | | | |
| | No | 18 (82) | 11 (91) | 6.35×10⁻¹ |
| | Yes | 4 (18) | 1 (9) | |
| Recurrence | | 1 (5) | 0 (0) | 1.00 |
| Fatal | | 0 (0) | 0 (0) | 1.00 |

### [Example 5: Identification of DNA Methylation Marker Capable of Detecting Low-Malignancy Endometrial Cancer]

A marker for discriminating high-malignancy group (YE-A) and low-malignancy group (YE-B) was explored. One hundred CpG sites, having the largest differences of the DNA methylation level between group (YE-A) and group (YE-B), were subjected to ROC analysis. ROC curve was drawn for the methylation level at each site, by plotting sensitivity versus specificity regarding discrimination between group (YE-A) and group (YE-B), and the area under the curve (AUC) was calculated.

From results of the ROC analysis, 68 CpG sites with an AUC of 0.95 or larger were found. Of these, 31 CpG sites were found to show increased methylation level in group (YE-A), whereas 37 CpG sites were found to show increased methylation level in group (YE-B) (Tables 10 and 11). Of such 68 CpG sites, 11 CpG sites were found to have an AUC of unity (100% sensitivity and specificity). Of 11 CpG sites, six sites were found to show increased methylation level in group (YE-A), whereas five CpG sites were found to show increased methylation level in group (YE-B). The methylation level at these 68 CpG sites was suggested to be useful as a marker that indicates risk and prognosis of juvenile endometrial cancer, and in particular to discriminate low malignancy (that is, non-poor prognosis) group. Since the patients with the low malignancy of juvenile endometrial cancer are expectable subjects for the preservation therapy, so that the methylation level at these CpG sites is also beneficial as a marker for discriminating endometrial cancer patients compatible to the preservation therapy.

**[Table 10]**

| Methylation level: YE-A > YE-B | | | |
|---|---|---|---|
| Chromosome No. | Chromosomal position | Gene name | AUC |
| 10 | 106400259 | (Non-coding) | 1.000 |
| 8 | 56852290 | LYN | 1.000 |
| 14 | 60973823 | (Non-coding) | 1.000 |
| 6 | 28226980 | ZKSCAN4 | 1.000 |
| 1 | 62660624 | L1TD1 | 1.000 |
| 6 | 10390919 | (Non-coding) | 1.000 |
| 13 | 112711380 | | 0.996 |
| 4 | 147561203 | POU4F2 | 0.996 |
| 6 | 29943464 | HCG9 | 0.992 |
| 6 | 10391237 | (Non-coding) | 0.992 |
| 5 | 140787623 | PCDHGA9 | 0.989 |
| | | PCDHGB5 | |
| | | PCDHGA8 | |
| | | PCDHGB4 | |
| | | PCDHGA7 | |
| | | PCDHGA6 | |
| | | PCDHGB3 | |
| | | PCDHGA5 | |
| | | PCDHGB2 | |
| | | PCDHGA4 | |
| | | PCDHGB1 | |
| | | PCDHGA3 | |
| | | PCDHGA2 | |
| | | PCDHGA1 | |
| 1 | 221050491 | HLX-AS1 | 0.989 |
| 6 | 27649120 | (Non-coding) | 0.988 |
| 19 | 52452447 | ZNF350-AS1 | 0.985 |
| 5 | 150326174 | ZNF300P1 | 0.981 |
| 6 | 10393778 | (Non-coding) | 0.977 |
| 18 | 70535389 | (Non-coding) | 0.977 |
| 4 | 111550830 | PITX2 | 0.973 |
| 13 | 112759355 | SOX1-OT | 0.970 |
| 7 | 35301188 | (Non-coding) | 0.962 |
| 14 | 29254530 | LINC02282 | 0.962 |
| 7 | 49815502 | VWC2 | 0.962 |
| 2 | 105459164 | LINC01158 | 0.958 |
| 1 | 91185422 | (Non-coding) | 0.955 |
| 4 | 41749443 | PHOX2B | 0.951 |
| 6 | 27647843 | (Non-coding) | 0.951 |

**[Table 11]**

| Methylation level: YE-B > YE-A | | | |
|---|---|---|---|
| Chromosome No. | Chromosomal position | Gene name | AUC |
| 8 | 132321917 | (Non-coding) | 1.000 |
| 11 | 5346249 | (Non-coding) | 1.000 |
| 21 | 31972506 | (Non-coding) | 1.000 |
| 2 | 1928480 | MYT1L | 1.000 |
| 1 | 248366399 | OR2M3 | 1.000 |
| 8 | 65549360 | CYP7B1 | 0.996 |
| 10 | 118138962 | CCDC172 | 0.996 |
| 8 | 114435951 | CSMD3 | 0.996 |
| 5 | 152981133 | GRIA1 | 0.996 |
| 16 | 5293539 | (Non-coding) | 0.992 |
| 2 | 119418938 | (Non-coding) | 0.992 |
| 14 | 82292196 | (Non-coding) | 0.992 |
| 12 | 59657344 | (Non-coding) | 0.992 |
| 11 | 107067568 | (Non-coding) | 0.992 |
| 11 | 6049230 | (Non-coding) | 0.992 |
| 2 | 2321788 | MYT1L | 0.992 |
| 11 | 5295849 | (Non-coding) | 0.989 |
| 1 | 152797107 | (Non-coding) | 0.989 |
| 5 | 166491296 | (Non-coding) | 0.985 |
| 5 | 166988043 | TENM2 | 0.985 |
| 10 | 18583838 | CACNB2 | 0.985 |
| 12 | 61961255 | (Non-coding) | 0.981 |
| 10 | 26461882 | MYO3A | 0.981 |
| 8 | 98376014 | LOC101927066 | 0.981 |
| 2 | 1984549 | MYT1L | 0.981 |
| 10 | 87364316 | GRID1-AS1 | 0.981 |
| 8 | 139114773 | (Non-coding) | 0.977 |
| 7 | 116151921 | (Non-coding) | 0.973 |
| 10 | 135030554 | KNDC1 | 0.972 |
| 12 | 132102188 | (Non-coding) | 0.970 |
| 2 | 1417164 | TPO | 0.968 |
| 8 | 72872845 | MSC-AS1 | 0.966 |
| 7 | 157933750 | PTPRN2 | 0.966 |
| 1 | 229706215 | (Non-coding) | 0.966 |
| 10 | 132461281 | (Non-coding) | 0.962 |
| 8 | 65528567 | CYP7B1 | 0.958 |
| 5 | 84126213 | (Non-coding) | 0.958 |
| 8 | 65525631 | CYP7B1 | 0.955 |
| 11 | 88911467 | TYR | 0.955 |
| 10 | 130844884 | (Non-coding) | 0.952 |
| 16 | 1088479 | (Non-coding) | 0.951 |
| 7 | 126746802 | GRM8 | 0.951 |

### [Example 6: Determination of Malignancy of Endometrial Cancer with DNA Methylation Marker]

For each of eleven CpG sites identified in Example 5 to have an AUC of unity, the methylation level at which the sum of sensitivity and specificity becomes maximum (approaching closest to the upper left corner on the graph of ROC curve) was determined to be a provisional diagnostic threshold value (cutoff value), on the basis of Youden's index. As illustrated in Fig. 3, group (YE-A) and group (YE-B) were discriminated with 100% sensitivity and specificity, for all of 11 CpG sites according to the set diagnostic threshold value. Hence, measurement of the methylation level at the CpG sites identified in Example 5 was suggested to enable determination of risk or prognosis of juvenile endometrial cancer, or discrimination of low malignancy (or, non-poor prognosis) group.

Moreover, since group (YE-A) and group (YE-B) of juvenile endometrial cancer patients are respectively contained in group (E-A) and group (E-B) of the whole endometrial cancer patients as demonstrated in Example 4, the DNA methylation marker identified in Example 5 was suggested to be usable as a marker that indicates risk or prognosis of cancer, not only for juvenile endometrial cancer patients but also for various types of endometrial cancer patients including senile endometrial cancer patients. Hence, for each of the DNA methylation level at eleven CpG sites identified in Example 5 to have an AUC of unity, an ROC curve was drawn by plotting sensitivity versus specificity regarding discrimination between high-malignancy group (E-A) and low-malignancy group (E-B), and the area under the curve (AUC) was calculated. Results are summarized in Table 12. Eleven CpG sites were found to have an AUC of approximately 0.94 to 0.99 or above, and half or more than half of them showed a specificity of 100%. Hence, these CpG sites were demonstrated to be useful as a marker that indicates risk or prognosis of cancer, for various types of endometrial cancer patients including juvenile endometrial cancer patients and senile endometrial cancer patients.

**[Table 12]**

| Chromosome No. | Chromosomal position | AUC | Youden | Sensitivity | Specificity |
|---|---|---|---|---|---|
| 10 | 106400259 | 0.9828 | 0.4052 | 0.8966 | 1.0000 |
| 8 | 56852290 | 0.9594 | 0.6019 | 0.8596 | 1.0000 |
| 14 | 60973823 | 0.9806 | 0.6095 | 0.9655 | 0.9375 |
| 6 | 28226980 | 0.9935 | 0.4900 | 0.9828 | 1.0000 |
| 1 | 62660624 | 0.9881 | 0.5795 | 0.9483 | 1.0000 |
| 6 | 10390919 | 0.9890 | 0.5044 | 0.9298 | 1.0000 |
| 8 | 132321917 | 0.9504 | 0.4489 | 0.8103 | 1.0000 |
| 11 | 5346249 | 0.9591 | 0.5281 | 0.9483 | 0.9375 |
| 21 | 31972506 | 0.9655 | 0.6206 | 0.9483 | 0.9375 |
| 2 | 1928480 | 0.9418 | 0.5580 | 0.8621 | 0.9375 |
| 1 | 248366399 | 0.9569 | 0.5059 | 0.9138 | 0.9375 |

## Claims

1. A method for detecting a cell or tissue derived from an endometrial cancer patient with poor prognosis, the method comprising:
detecting DNA methylation level at a target CpG site in a genomic DNA derived from an endometrial cancer cell or tissue containing the cell; and
detecting a cell or tissue derived from the endometrial cancer patient with poor prognosis, on the basis of the detected DNA methylation level,
wherein the target CpG site is at least one CpG site selected from the group consisting of CpG sites located at or near the chromosomal positions listed in Table A.
**[Table A]**
| CpG site | | | | | |
|---|---|---|---|---|---|
| Chromosome No. | Chromosomal position | Chromosome No. | Chromosomal position | Chromosome No. | Chromosomal position |
| 10 | 106400259 | 13 | 112759355 | 10 | 18583838 |
| 8 | 56852290 | 7 | 35301188 | 12 | 61961255 |
| 14 | 60973823 | 14 | 29254530 | 10 | 26461882 |
| 6 | 28226980 | 7 | 49815502 | 8 | 98376014 |
| 1 | 62660624 | 2 | 105459164 | 2 | 1984549 |
| 6 | 10390919 | 1 | 91185422 | 10 | 87364316 |
| 8 | 132321917 | 4 | 41749443 | 8 | 139114773 |
| 11 | 5346249 | 6 | 27647843 | 7 | 116151921 |
| 21 | 31972506 | 8 | 65549360 | 10 | 135030554 |
| 2 | 1928480 | 10 | 118138962 | 12 | 132102188 |
| 1 | 248366399 | 8 | 114435951 | 2 | 1417164 |
| 13 | 112711380 | 5 | 152981133 | 8 | 72872845 |
| 4 | 147561203 | 16 | 5293539 | 7 | 157933750 |
| 6 | 29943464 | 2 | 119418938 | 1 | 229706215 |
| 6 | 10391237 | 14 | 82292196 | 10 | 132461281 |
| 5 | 140787623 | 12 | 59657344 | 8 | 65528567 |
| 1 | 221050491 | 11 | 107067568 | 5 | 84126213 |
| 6 | 27649120 | 11 | 6049230 | 8 | 65525631 |
| 19 | 52452447 | 2 | 2321788 | 11 | 88911467 |
| 5 | 150326174 | 11 | 5295849 | 10 | 130844884 |
| 6 | 10393778 | 1 | 152797107 | 16 | 1088479 |
| 18 | 70535389 | 5 | 166491296 | 7 | 126746802 |
| 4 | 111550830 | 5 | 166988043 | | |

2. A method for detecting a cell or tissue derived from an endometrial cancer patient compatible to preservation therapy, the method comprising:
detecting DNA methylation level at a target CpG site in a genomic DNA derived from an endometrial cancer cell or tissue containing the cell; and
detecting a cell or tissue derived from the endometrial cancer patient compatible to preservation therapy, on the basis of the detected DNA methylation level,
wherein the target CpG site is at least one CpG site selected from the group consisting of CpG sites located at or near the chromosomal positions listed in Table A.
**[Table A]**
| CpG site | | | | | |
|---|---|---|---|---|---|
| Chromosome No. | Chromosomal position | Chromosome No. | Chromosomal position | Chromosome No. | Chromosomal position |
| 10 | 106400259 | 13 | 112759355 | 10 | 18583838 |
| 8 | 56852290 | 7 | 35301188 | 12 | 61961255 |
| 14 | 60973823 | 14 | 29254530 | 10 | 26461882 |
| 6 | 28226980 | 7 | 49815502 | 8 | 98376014 |
| 1 | 62660624 | 2 | 105459164 | 2 | 1984549 |
| 6 | 10390919 | 1 | 91185422 | 10 | 87364316 |
| 8 | 132321917 | 4 | 41749443 | 8 | 139114773 |
| 11 | 5346249 | 6 | 27647843 | 7 | 116151921 |
| 21 | 31972506 | 8 | 65549360 | 10 | 135030554 |
| 2 | 1928480 | 10 | 118138962 | 12 | 132102188 |
| 1 | 248366399 | 8 | 114435951 | 2 | 1417164 |
| 13 | 112711380 | 5 | 152981133 | 8 | 72872845 |
| 4 | 147561203 | 16 | 5293539 | 7 | 157933750 |
| 6 | 29943464 | 2 | 119418938 | 1 | 229706215 |
| 6 | 10391237 | 14 | 82292196 | 10 | 132461281 |
| 5 | 140787623 | 12 | 59657344 | 8 | 65528567 |
| 1 | 221050491 | 11 | 107067568 | 5 | 84126213 |
| 6 | 27649120 | 11 | 6049230 | 8 | 65525631 |
| 19 | 52452447 | 2 | 2321788 | 11 | 88911467 |
| 5 | 150326174 | 11 | 5295849 | 10 | 130844884 |
| 6 | 10393778 | 1 | 152797107 | 16 | 1088479 |
| 18 | 70535389 | 5 | 166491296 | 7 | 126746802 |
| 4 | 111550830 | 5 | 166988043 | | |

3. A method for determining malignant transformation risk of endometrium, the method comprising:
detecting DNA methylation level at a target CpG site in a genomic DNA derived from an endometrial cell or tissue containing the cell; and
determining malignant transformation risk in the endometrium, on the basis of the detected DNA methylation level,
wherein the target CpG site is at least one CpG site selected from the group consisting of CpG sites located at or near the chromosomal positions listed in Table A.
**[Table A]**
| CpG site | | | | | |
|---|---|---|---|---|---|
| Chromosome No. | Chromosome position | Chromosome No. | Chromosome position | Chromosome No. | Chromosomal position |
| 10 | 106400259 | 13 | 112759355 | 10 | 18583838 |
| 8 | 56852290 | 7 | 35301188 | 12 | 61961255 |
| 14 | 60973823 | 14 | 29254530 | 10 | 26461882 |
| 6 | 28226980 | 7 | 49815502 | 8 | 98376014 |
| 1 | 62660624 | 2 | 105459164 | 2 | 1984549 |
| 6 | 10390919 | 1 | 91185422 | 10 | 87364316 |
| 8 | 132321917 | 4 | 41749443 | 8 | 139114773 |
| 11 | 5346249 | 6 | 27647843 | 7 | 116151921 |
| 21 | 31972506 | 8 | 65549360 | 10 | 135030554 |
| 2 | 1928480 | 10 | 118138962 | 12 | 132102188 |
| 1 | 248366399 | 8 | 114435951 | 2 | 1417164 |
| 13 | 112711380 | 5 | 152981133 | 8 | 72872845 |
| 4 | 147561203 | 16 | 5293539 | 7 | 157933750 |
| 6 | 29943464 | 2 | 119418938 | 1 | 229706215 |
| 6 | 10391237 | 14 | 82292196 | 10 | 132461281 |
| 5 | 140787623 | 12 | 59657344 | 8 | 65528567 |
| 1 | 221050491 | 11 | 107067568 | 5 | 84126213 |
| 6 | 27649120 | 11 | 6049230 | 8 | 65525631 |
| 19 | 52452447 | 2 | 2321788 | 11 | 88911467 |
| 5 | 150326174 | 11 | 5295849 | 10 | 130844884 |
| 6 | 10393778 | 1 | 152797107 | 16 | 1088479 |
| 18 | 70535389 | 5 | 166491296 | 7 | 126746802 |
| 4 | 111550830 | 5 | 166988043 | | |

4. The method according to any one of claims 1 to 3, wherein the target CpG site is at least one CpG site selected from the group consisting of CpG sites located at chromosome 10, position 106,400,259; chromosome 8, position 56,852,290; chromosome 14, position 60,973,823; chromosome 6, position 28,226,980; chromosome 1, position 62,660,624; chromosome 6, position 10,390,919; chromosome 8, position 132,321,917; chromosome 11, position 5,346,249; chromosome 21, position 31,972,506; chromosome 2, position 1,928,480; and chromosome 1, position 248,366,399, and CpG sites located near the positions.

5. The method according to any one of claims 1 to 3, wherein the target CpG site is at least one CpG site selected from the group consisting of:
CpG site contained in the DNA region of chromosome 10, from position 106,400,199 to position 106,400,320;
CpG site contained in the region of chromosome 8, from position 56,852,230 to position 56,852,351;
CpG site contained in the region of chromosome 14, from position 60,973,763 to position 60,973,884;
CpG site contained in the region of chromosome 6, from position 28,226,920 to position 28,227,041;
CpG site contained in the region of chromosome 1, from position 62,660,564 to position 62,660,685;
CpG site contained in the region of chromosome 6, from position 10,390,859 to position 10,390,980;
CpG site contained in the region of chromosome 8, from position 132,321,857 to position 132,321,978;
CpG site contained in the region of chromosome 11, from position 5,346,189 to position 5,346,310;
CpG site contained in the region of chromosome 21, from position 31,972,446 to position 31,972,567;
CpG site contained in the region of chromosome 2, from position 1,928,420 to position 1,928,541; and
CpG site contained in the region of chromosome 1, from position 248,366,339 to position 248,366,460.

6. The method according to any one of claims 1 to 3, wherein the target CpG site is at least one CpG site selected from the group consisting of CpG sites contained in a DNA region consisting of any one of nucleotide sequences represented by SEQ ID NOs 1 to 11 or complementary sequences thereof.

7. The method according to any one of claims 1 to 6, wherein the detection of the DNA methylation level comprises detecting the DNA methylation level at the target CpG site, by using the genomic DNA which has been treated with bisulfite.

8. The method according to claim 7, wherein the DNA methylation level is detected by pyrosequencing, mass spectrometry, bead array method, or ion exchange chromatography.

9. A primer or a probe for use in determining prognosis of endometrial cancer, determining an endometrial cancer patient compatible to preservation therapy, or determining risk of endometrial cancer,
wherein the primer or the probe has at least 12 base long, and is hybridizable with a target CpG site which has been treated with bisulfite, and
the target CpG site is at least one CpG site selected from the group consisting of CpG sites located at or near the chromosomal positions listed in Table A.
**[Table A]**
| CpG site | | | | | |
|---|---|---|---|---|---|
| Chromosome No. | Chromosomal position | Chromosome No. | Chromosomal position | Chromosome No. | Chromosomal position |
| 10 | 106400259 | 13 | 112759355 | 10 | 18583838 |
| 8 | 56852290 | 7 | 35301188 | 12 | 61961255 |
| 14 | 60973823 | 14 | 29254530 | 10 | 26461882 |
| 6 | 28226980 | 7 | 49815502 | 8 | 98376014 |
| 1 | 62660624 | 2 | 105459164 | 2 | 1984549 |
| 6 | 10390919 | 1 | 91185422 | 10 | 87364316 |
| 8 | 132321917 | 4 | 41749443 | 8 | 139114773 |
| 11 | 5346249 | 6 | 27647843 | 7 | 116151921 |
| 21 | 31972506 | 8 | 65549360 | 10 | 135030554 |
| 2 | 1928480 | 10 | 118138962 | 12 | 132102188 |
| 1 | 248366399 | 8 | 114435951 | 2 | 1417164 |
| 13 | 112711380 | 5 | 152981133 | 8 | 72872845 |
| 4 | 147561203 | 16 | 5293539 | 7 | 157933750 |
| 6 | 29943464 | 2 | 119418938 | 1 | 229706215 |
| 6 | 10391237 | 14 | 82292196 | 10 | 132461281 |
| 5 | 140787623 | 12 | 59657344 | 8 | 65528567 |
| 1 | 221050491 | 11 | 107067568 | 5 | 84126213 |
| 6 | 27649120 | 11 | 6049230 | 8 | 65525631 |
| 19 | 52452447 | 2 | 2321788 | 11 | 88911467 |
| 5 | 150326174 | 11 | 5295849 | 10 | 130844884 |
| 6 | 10393778 | 1 | 152797107 | 16 | 1088479 |
| 18 | 70535389 | 5 | 166491296 | 7 | 126746802 |
| 4 | 111550830 | 5 | 166988043 | | |

10. The primer or the probe according to claim 9, wherein the target CpG site is at least one CpG site selected from the group consisting of CpG sites located at chromosome 10, position 106,400,259; chromosome 8, position 56,852,290; chromosome 14, position 60,973,823; chromosome 6, position 28,226,980; chromosome 1, position 62,660,624; chromosome 6, position 10,390,919; chromosome 8, position 132,321,917; chromosome 11, position 5,346,249; chromosome 21, position 31,972,506; chromosome 2, position 1,928,480; and chromosome 1, position 248,366,399, and CpG sites located near the positions.

11. The primer or the probe according to claim 9, wherein the target CpG site is at least one CpG site selected from the group consisting of the following CpG sites:
CpG site contained in the region of chromosome 10, from position 106,400,199 to position 106,400,320;
CpG site contained in the region of chromosome 8, from position 56,852,230 to position 56,852,351;
CpG site contained in the region of chromosome 14, from position 60,973,763 to position 60,973,884;
CpG site contained in the region of chromosome 6, from position 28,226,920 to position 28,227,041;
CpG site contained in the region of chromosome 1, from position 62,660,564 to position 62,660,685;
CpG site contained in the region of chromosome 6, from position 10,390,859 to position 10,390,980;
CpG site contained in the region of chromosome 8, from position 132,321,857 to position 132,321,978;
CpG site contained in the region of chromosome 11, from position 5,346,189 to position 5,346,310;
CpG site contained in the region of chromosome 21, from position 31,972,446 to position 31,972,567;
CpG site contained in the region of chromosome 2, from position 1,928,420 to position 1,928,541; and
CpG site contained in the region of chromosome 1, from position 248,366,339 to position 248,366,460.

12. The primer or the probe according to claim 9, wherein the target CpG site is at least one CpG site selected from the group consisting of CpG sites contained in a DNA region consisting of any one of nucleotide sequences represented by SEQ ID NOs 1 to 11 or complementary sequences thereof.
